# EUROPEAN PATENT APPLICATION

(11) **EP 4 785 881 A1**
(43) Date of publication of application: **05.08.2026**
(21) Application number: 24869587.6
(22) Date of filing: 21.02.2024
(51) Int. Cl.: A61B 17/32

(54) **ULTRASONIC SURGICAL TOOL, MOUNTABLE ULTRASONIC SURGICAL TOOL ASSEMBLY AND SURGICAL ROBOT SYSTEM**

(30) Priority: 26.09.2023 CN 202311251898; 27.10.2023 CN 202311406969
(71) Applicant: Beijing Surgerii Robotics Company Limited, Beijing 100192 (CN)
(72) Inventor: XU, Kai, 100192 Beijing (CN); DING, Yue, 100192 Beijing (CN)
(74) Representative: karo IP
(86) International application number: PCT/CN2024/077926
(87) International publication number: WO 2025/066015

(57) **Abstract**

The present disclosure relates to the field of medical instruments. Disclosed are an ultrasonic surgical tool, an assemblable ultrasonic surgical tool assembly and a surgical robot system. The ultrasonic surgical tool comprises: an ultrasonic blade assembly, disposed at a distal end of the ultrasonic surgical tool, the ultrasonic blade assembly comprising an ultrasonic blade; an ultrasonic transducer, coupled to a proximal end of the ultrasonic blade to output vibration to the ultrasonic blade; and a clamping assembly, connected with the ultrasonic blade assembly, configured to mount or demount the ultrasonic surgical tool. The present disclosure is conducive to enhancing the output power of ultrasonic surgical tools.

## Description

### CROSS REFERENCES TO RELATED APPLICATIONS

The present application claims the benefit of priority to the Chinese patent application filed on September 26, 2023 with the application number 2023112518985 and titled "Surgical Tool Assembly and Surgical Robot System", the Chinese patent application filed on October 27, 2023 with the application number 2023114069694 and titled "Ultrasonic Surgical Tool, Assemblable Ultrasonic Surgical Tool Assembly and Surgical Robot System", the entire contents of which are incorporated herein by reference in their entirety.

### TECHNICAL FIELD

The present disclosure relates to the field of medical instruments, and particularly to an ultrasonic surgical tool, an assemblable ultrasonic surgical tool assembly and a surgical robot system.

### BACKGROUND

The laparoscopic operation is the operation mode developed gradually and widely used in recent years, and it has the advantages of small wound and greatly reduces the rehabilitation time, discomfort experience and side effect after healing. Laparoscopic operation, especially single-port laparoscopic operation, performed by surgical robots can optimize the surgical form through computer remote control technology.

Ultrasonic blade is an energy surgical tool widely used in operation. The ultrasonic transducer of the ultrasonic blade converts the received high-frequency alternating voltage into high-frequency vibration and transmits the high-frequency vibration to the head of the ultrasonic blade. The high-frequency vibrating ultrasonic blade head is contacted with the tissue so as to cut and coagulate the tissue.

The ultrasonic blade used by the surgical robot system for laparoscopic operation often has the problem of insufficient output power, which affects the efficiency of tissue cutting.

### SUMMARY OF THE INVENTION

In some embodiments, the present disclosure provides an ultrasonic surgical tool comprising:
an ultrasonic blade assembly, disposed at a distal end of the ultrasonic surgical tool, the ultrasonic blade assembly comprising an ultrasonic blade;
an ultrasonic transducer, coupled to a proximal end of the ultrasonic blade to output vibration to the ultrasonic blade; and
a clamping assembly, connected with the ultrasonic blade assembly, configured to mount or demount the ultrasonic surgical tool.

In some embodiments, the present disclosure provides an assemblable ultrasonic surgical tool assembly, comprising:
the ultrasonic surgical tool according to any one of claims 1 to 12; and
an auxiliary surgical instrument, detachably connected with the ultrasonic surgical tool, configured to drive the ultrasonic surgical tool to move, the auxiliary surgical instrument comprising:
   an instrument stem; and
   an instrument end effector, disposed at a distal end of the instrument stem, the instrument end effector being detachably connected with the clamping assembly of the ultrasonic surgical tool.

In some embodiments, the present disclosure provides a surgical robot system, comprising:
an operation trolley, comprising at least one robotic arm; and
the assemblable ultrasonic surgical tool assembly according to any one of claims 13 to 19, wherein the auxiliary surgical instrument of the assemblable ultrasonic surgical tool assembly is disposed at a distal end of the at least one robotic arm.

### BRIEF DESCRIPTION OF THE DRAWINGS

In order to explain the technical solutions in the embodiments of the present disclosure more clearly, the accompanying drawings used in the description of the embodiments of the present disclosure will be briefly introduced below. The accompanying drawings in the following description only show some of the embodiments of the present disclosure, and for those of ordinary skill in the art, other embodiments would also have been obtained from the contents of the embodiments of the present disclosure and these accompanying drawings without involving any inventive effort.
FIG. 1A shows a cross-sectional schematic diagram of part of the structure of an ultrasonic surgical tool according to some embodiments of the present disclosure;
FIG. 1B shows a structural schematic diagram of part of the structure of an ultrasonic surgical tool according to some embodiments of the present disclosure;
FIG. 2 shows a structural schematic diagram of part of the structure of an auxiliary surgical instrument according to some embodiments of the present disclosure;
FIG. 3 shows a schematic diagram of a surgical robot system according to some embodiments of the present disclosure;
FIG. 4A shows a structural schematic diagram of another part of the structure of an ultrasonic surgical tool according to some embodiments of the present disclosure;
FIG. 4B shows a cross-sectional schematic diagram of another part of the structure of an ultrasonic surgical tool according to some embodiments of the present disclosure;
FIG. 5 shows a structural schematic diagram of an ultrasonic surgical tool according to some embodiments of the present disclosure;
FIG. 6 shows an amplitude distribution of an ultrasonic surgical tool according to some embodiments of the present disclosure;
FIG. 7A shows a structural schematic diagram of a clamping assembly according to some embodiments of the present disclosure;
FIG. 7B shows a structural schematic diagram of a clamping assembly according to some embodiments of the present disclosure in another state;
FIG. 8A shows a structural schematic diagram of yet another part of the structure of an ultrasonic surgical tool according to some embodiments of the present disclosure;
FIG. 8B shows a structural schematic diagram of yet another part of the structure of an ultrasonic surgical tool according to some embodiments of the present disclosure at another angle;
FIG. 9A shows a schematic diagram of the connection ralationship between an ultrasonic surgical tool and an auxiliary surgical instrument according to some embodiments of the present disclosure;
FIG. 9B shows a structural schematic diagram of part of the structure of an ultrasonic surgical tool and an auxiliary surgical instrument according to some embodiments of the present disclosure;
FIG. 10A shows a structural schematic diagram in which the jaw in an assemblable ultrasonic surgical tool assembly according to some embodiments of the present disclosure is in an open state;
FIG. 10B shows a structural schematic diagram in which the jaw in an assemblable ultrasonic surgical tool assembly according to some embodiments of the present disclosure is in a closed state;
FIG. 11A shows a structural schematic diagram of an auxiliary surgical instrument according to some embodiments of the present disclosure;
FIG. 11B shows a structural schematic diagram of the first continuum structure of an instrument stem according to some embodiments of the present disclosure;
FIG. 12 shows a structural schematic diagram of a driving device according to some embodiments of the present disclosure;
FIG. 13 shows a schematic diagram of a surgical robot system according to another embodiment of the present disclosure.

### DETAILED DESCRIPTION

To make the solved technical problems, used technical solutions, and achieved technical effects of the present disclosure more clearly, the technical solutions of the embodiments of the present disclosure will be further described in detail below with reference to the accompanying drawings. Obviously, the described embodiments are only exemplary embodiments, but not all of embodiments, of the present disclosure.

In the description of the present disclosure, it should be noted that, orientational or positional relationships indicated by the terms "center", "upper", "lower", "left", "right", "vertical", "horizontal", "inner", "outer" and the like are the orientational or positional relationships shown based on the accompanying drawings, and are only for ease of describing the present disclosure and simplifying the description, rather than indicating or implying that the apparatus or element referred to must have a specific orientation or be constructed and operated in a specific orientation, and therefore cannot be construed as limiting the present disclosure. In addition, the terms "first" and "second" are used for descriptive purposes only, and cannot be understood as indicating or implying relative importance.

In the description of the present disclosure, it should be noted that, unless otherwise specified and defined, the term "mount", "connected", and "connect", or "couple" should be comprehended in a broad sense. For example, the term may be a fixed connection or a detachable connection; or may be a mechanical connection or an electrical connection; may be a direct connection or an indirect connection via an intermediate medium; or may be internal communication between two elements. For those of ordinary skill in the art, specific meanings of the foregoing terms in the present disclosure may be understood based on specific situations. In the present disclosure, an end close to an operator (e.g., a surgeon) is defined as a proximal end, a proximal portion, or a rear end, a rear portion, an end close to a patient is defined as a distal end, a distal portion, or a front end, a front portion. It may be understood by those skilled in the art that the embodiments of the present disclosure may be used for a medical instrument or a surgical robot, and may also be used for other non-medical apparatus.

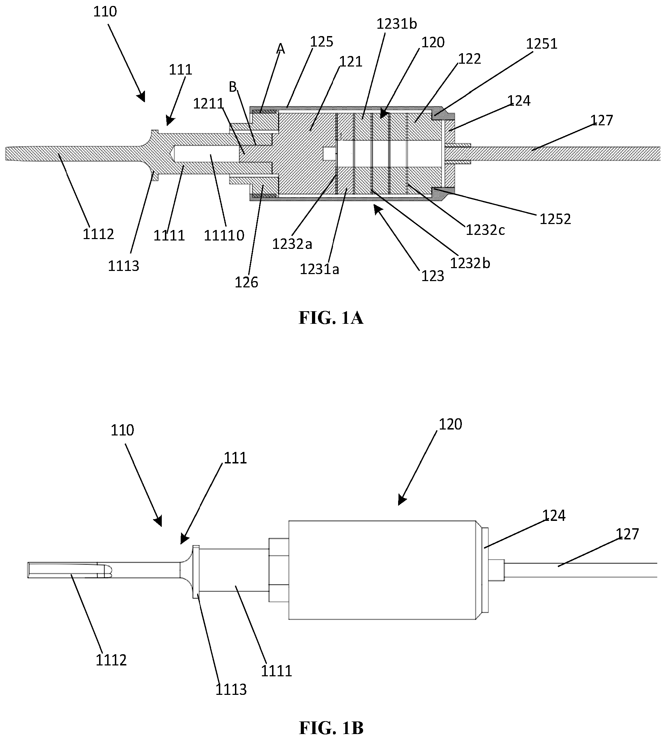

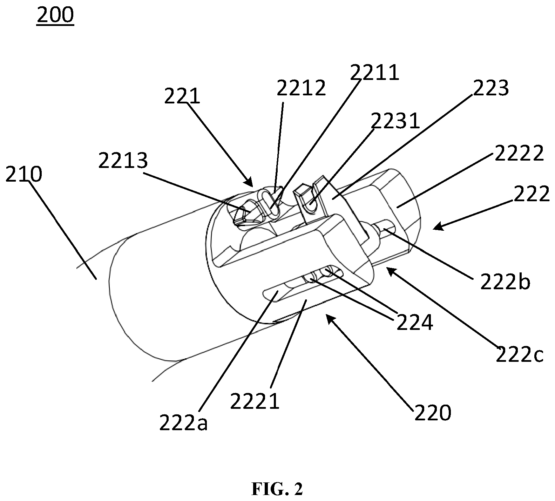

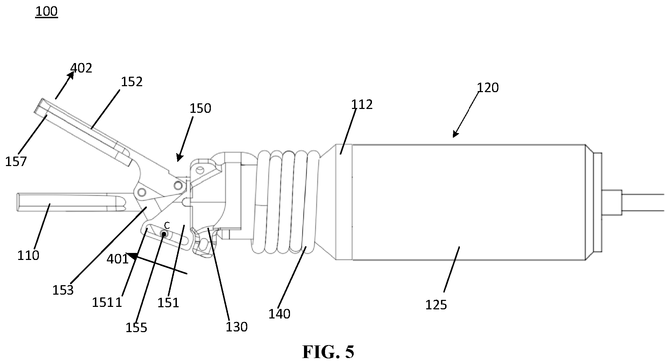

FIG. 1A shows a cross-sectional schematic diagram of part of the structure of an ultrasonic surgical tool 100 according to some embodiments of the present disclosure.FIG. 1B shows a structural schematic diagram of part of the structure of an ultrasonic surgical tool 100 according to some embodiments of the present disclosure.FIG. 2 shows a structural schematic diagram of part of the structure of an auxiliary surgical instrument 200 according to some embodiments of the present disclosure.FIG. 5 shows a structural schematic diagram of an ultrasonic surgical tool 100 according to some embodiments of the present disclosure. In some embodiments, the ultrasonic surgical tool 100 and the auxiliary surgical instrument 200 can be assembled to form an assemblable ultrasonic surgical tool assembly. In some embodiments, the assemblable ultrasonic surgical tool assembly which is assembled from the ultrasonic surgical tool 100 and the auxiliary surgical instrument 200 may be used in a surgical robot system. The auxiliary surgical instrument 200 of the assemblable ultrasonic surgical tool assembly, can be disposed at the distal end of a robotic arm of a surgical robot system, such as a positioning arm of a surgical robot system. The auxiliary surgical instrument 200 can be moved under the control of a user.

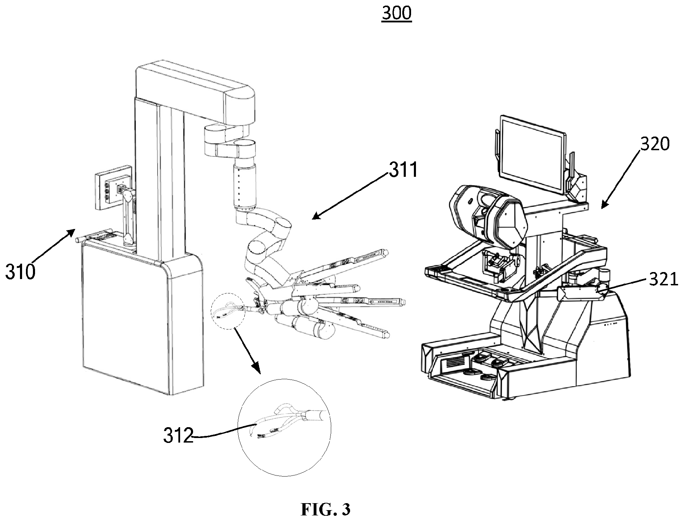

The surgical robot system may be a variety of suitable surgical robot systems including laparoscopic surgical robot systems. FIG. 3 shows a schematic diagram of a surgical robot system 300 according to some embodiments of the present disclosure. As shown in FIG. 3, the surgical robot system 300 may comprise an operation trolley 310 and a surgeon console 320. The operation trolley 310 may comprise at least one robotic arm 311. The surgeon console 320 may comprise at least one master manipulator 321. As shown in FIG. 3, at least one robotic arm 311 can be movably disposed on the operation trolley 310. In some embodiments, at least one robotic arm 311 may be a positioning arm of a surgical robot system, and the distal end of the at least one robotic arm 311 may carry at least one surgical instrument 312 (e.g., a clamp, a curved scissors, an endoscope, an auxiliary surgical instrument 200, or the like). At least one master manipulator 321 is provided on the surgeon console 320 for receiving the operation of the user to the at least one master manipulator 321. The surgeon console 320 may communicate and connect with the operation trolley 310. In an operation, the operation trolley 310 is usually positioned at the patient's side, and a user can issue control commands by operating at least one master manipulator 321 of the surgeon console 320, to control at least one surgical instrument 312 carried by the operation trolley 310 to perform surgical procedures on the patient.

As shown in FIG. 1A or FIG. 1B, the ultrasonic surgical tool 100 may comprise an ultrasonic blade assembly 110 and an ultrasonic transducer 120. The ultrasonic surgical tool 100 may also comprise a clamping assembly (not shown in Figs. 1A and 1B). As shown in FIG. 1A or FIG. 1B, the ultrasonic blade assembly 110 is disposed at a distal end of the ultrasonic surgical tool 100 and comprises an ultrasonic blade 111. The ultrasonic transducer 120 may be coupled to a proximal end of the ultrasonic blade 111 to output vibrations to the ultrasonic blade 111.

In some embodiments, the ultrasonic blade 111 may be an amplitude bar with a reduced transverse dimension. In some embodiments, the ultrasonic blade 111 may comprise a proximal section 1111 and a distal section 1112. As shown in FIG. 1A or FIG. 1B, the proximal section 1111 is coupled to the ultrasonic transducer 120, and in the ultrasonic surgical tool 100, high-frequency vibration is transmitted to the proximal section 1111 of the ultrasonic blade 111 from the ultrasonic transducer 120 and then to the distal section 1112 of the ultrasonic blade 111. In the operation, the distal section 1112 is in contact with biological tissue to perform surgical procedures such as cutting and coagulation to the biological tissue. As shown in FIG. 1A or FIG. 1B, a transverse dimension of the distal section 1112 may be less than a transverse dimension of the proximal section 1111. The amplitude in the ultrasonic blade can be amplified well by the ultrasonic blade with reduced transverse dimension, which is good for improving the operation efficiency. It may be understood by those skilled in the art that the shape of the ultrasonic blade 111 is not limited to the above-mentioned shape, it may be an ultrasonic blade with a longitudinal cross-section edge of an exponential curve or a rounded step or slope, and it may also be a conical or stepped ultrasonic blade. In some embodiments, the axis of the horizontal cross section of the distal section 1112 of the ultrasonic blade 111 may be a curved arc to facilitate performing surgical procedures through the ultrasonic blade 111. In some embodiments, the distal section 1112 of the ultrasonic blade 111 may comprise at least one cutting edge structure. The cutting edge structure is helpful for enhancing the mechanical cutting effect of the ultrasonic blade head to the biological tissue, improving the cutting speed of the surgical tool to the biological tissue, besides, it has the advantage of low processing and manufacturing cost. In some embodiments, the ultrasonic blade 111 may be a half-wavelength ultrasonic blade. It may be understood by those skilled in the art that the length of the half-wavelength ultrasonic blade may be half the wavelength of the mechanical harmonic transmitted in the ultrasonic blade. In some embodiments, the ultrasonic blade 111 may be a 55 kHz ultrasonic blade.

In some embodiments, as shown in FIG. 1A, the ultrasonic transducer 120 may comprise a front plate 121, a rear plate 122, and an ultrasonic transducer section 123. The front plate 121 is disposed at the distal end of the ultrasonic transducer 120 and the rear plate 122 is disposed at the proximal end of the ultrasonic transducer 120. The ultrasonic transducer section 123 may be disposed between the front plate 121 and the rear plate 122. The ultrasonic transducer section 123 may comprise a plurality of piezoelectric ceramic plates (e.g., piezoelectric ceramic plates 1231a, 1231b, etc. shown in FIG. 1A) and a plurality of electrode slices (e.g., electrode slices 1232a, 1232b, 1232c, etc. shown in FIG. 1A). As shown in FIG. 1A, a plurality of electrode slices may be disposed between the front plate 121 and the ultrasonic transducer section 123 (e.g., electrode slice 1232a), between the plurality of piezoelectric ceramic plates (e.g., electrode slice 1232b) and between the ultrasonic transducer section 123 and the rear plate 122 (e.g., electrode slice 1232c).

In some embodiments, the plurality of electrode slices may be copper electrode slices. In some embodiments, the thickness of each of the plurality of electrode slices may be 0.2 mm. In some embodiments, the ultrasonic transducer 120 may also comprise a plurality of wires, the distal ends of the plurality of wires being connected to a plurality of electrode slices (e.g., electrode slices 1232a, 1232b, 1232c, etc. shown in FIG. 1A), respectively. As shown in FIG. 1A or FIG. 1B, a plurality of wires may be constrained to a wire bundle 127 at the proximal end of the ultrasonic transducer 120. The proximal ends of the plurality of wires (or wire bundle 127) may be connected to a power source, which may be disposed in the surgical robot system 300. In some embodiments, the ultrasonic transducer 120 may also comprise an interface (not shown). The interface is disposed at the proximal end of the plurality of wires, and the plurality of wires may be connected to a power source via the interface, thereby facilitating the improvement of the convenience of connecting the ultrasonic surgical tool 100 to the power source. Therefore, it is convenient to adjust the position of the ultrasonic surgical tool 100.

The power source applies high-frequency voltage to the plurality of electrode slices through plurality of wires, and the multiple piezoelectric ceramic plates perform high-frequency vibration along the thickness direction due to the inverse piezoelectric effect. The high-frequency vibration is transmitted to the ultrasonic blade 111. The high-frequency vibrating ultrasonic blade 111 can cut or coagulate the biological tissue when it is in contact with the biological tissue. In some embodiments, each of the plurality of piezoelectric ceramic plates may be a piezoelectric ceramic plate having an outer diameter dimension of 12 mm, an inner diameter dimension of 4 mm, and a thickness of 2.5 mm. The plurality of piezoelectric ceramic plates may comprise a central through hole for the plurality of wires to pass through. The plurality of electrode slices may comprise a central through hole for the plurality of wires to pass through.

In some embodiments, the front plate 121 may be made of a low acoustic impedance material such as titanium alloy, aluminum alloy, or the like, so as to achieve a higher output amplitude. The rear plate 122 may be made of a high acoustic impedance material such as stainless steel, tungsten alloy, or the like, so as to reduce the amplitude of the proximal end surface of the ultrasonic transducer 120. In some embodiments, the ultrasonic transducer 120 may be a half-wavelength ultrasonic transducer. It may be understood by those skilled in the art that the length of the half-wavelength ultrasonic transducer may be half the wavelength of the mechanical harmonic transmitted in the ultrasonic transducer. In some embodiments, the ultrasonic transducer 120 may be a full-wavelength ultrasonic transducer. The length of the full-wavelength ultrasonic transducer may be the wavelength of the mechanical harmonic transmitted in the ultrasonic transducer. In some embodiments, the ultrasonic transducer 120 may be a 55 kHz ultrasonic transducer.

In some embodiments, the ultrasonic transducer 120 may also comprise an ultrasonic transducer shell 125 and a fastener 126. As shown in FIG. 1A, the ultrasonic transducer shell 125 may be sleeved on the front plate 121, the ultrasonic transducer section 123 and the rear plate 122. The ultrasonic transducer shell 125 may comprise a bump (e.g., bump 1251 and/or 1252 shown in FIG. 1A) disposed at a proximal end of the ultrasonic transducer shell 125 and propped against a proximal end of the rear plate 122. In some embodiments, the inner circumferential surface of the ultrasonic transducer shell 125 may comprise a threaded structure, the outer circumferential surface of the fastener 126 may comprise a threaded structure, and the outer circumferential surface of the fastener 126 may be in threaded connection with the inner circumferential surface of the ultrasonic transducer shell 125 (e.g., at the connection as indicated by A in FIG. 1A, a threaded connection is made). As shown in FIG. 1A, a proximal end of the fastener 126 may be propped against a distal end of the front plate 121. Based on the fastener 126 and the bumps 1251 and 1252 disposed at the proximal end of the ultrasonic transducer shell 125, a plurality of piezoelectric ceramic plates comprised in the ultrasonic transducer section 123 (e.g., the piezoelectric ceramic plate 1231a, 1231b, etc. shown in FIG. 1A) and a plurality of electrode slices (e.g., electrode slices 1232a, 1232b, 1232c, etc. shown in FIG. 1A) can be compressed.

In some embodiments, as shown in FIG. 1A or FIG. 1B, the ultrasonic transducer 120 may also comprise a pad 124. The pad 124 may be disposed at the proximal end of the ultrasonic transducer 120, in sealing connection with the proximal end of the ultrasonic transducer shell 125. In some embodiments, the pad 124 and the ultrasonic transducer shell 125 may be sealingly connected by gluing or the like. Therefore, the inner holes of the plurality of piezoelectric ceramic plates and the plurality of electrode slices comprised in the ultrasonic transducer 120 can be sealed. On one hand, it can avoid the plurality of piezoelectric ceramic plates and the plurality of electrode slices from being polluted. On the other hand, it can avoid affecting the electric connection of the electrode slices, therefore, it is advantageous to improve the safety of the ultrasonic surgical tool 100. The pad 124 may comprise a central through hole for the plurality of wires to pass through and to hold the plurality of wires. Based on the pad 124, the plurality of wires can be clamped, which is conducive to avoiding the pulling of the parts of the the plurality of wires connected in the ultrasonic transducer 120 during the control of the movement of the ultrasonic surgical tool 100, thereby enhancing the stability of the ultrasonic surgical tool 100.

As shown in FIG. 1A, the proximal section 1111 of the ultrasonic blade 111 comprises a blind hole 11110 located on a central axis of the proximal section 1111. The front plate 121 comprises a columnar structure 1211 disposed at the distal end of the front plate 121 and located on a central axis of the front plate 121. As shown in FIG. 1A, at least a portion of the proximal section 1111 of the ultrasonic blade 111 extends into the fastener 126. In some embodiments, an inner circumferential surface of the blind hole 11110 of the proximal section 1111 of the ultrasonic blade 111 may comprise a threaded structure, and an outer circumferential surface of the columnar structure 1211 of the proximal end of the front plate 121 may comprise a threaded structure. The inner circumferential surface of the blind hole 11110 may be in threaded connection with the outer circumferential surface of the columnar structure 1211 (e.g., in threaded connection at B as shown in FIG. 1A).

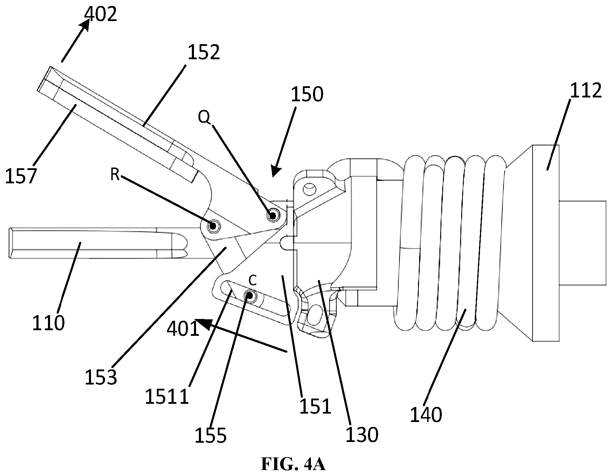

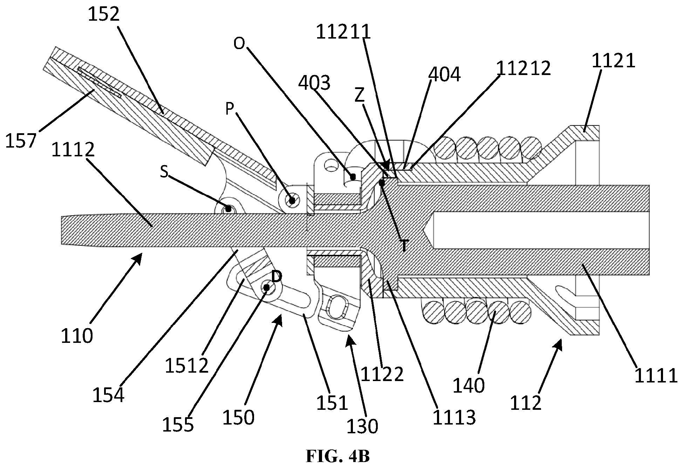

In some embodiments, the ultrasonic blade 111 is detachably connected to the front plate 121. It may be understood by those skilled in the art that the detachable connection between the ultrasonic blade 111 and the front plate 121 is not limited to the threaded connection, but may be any suitable connection, such as, for example, by means of inter-fitting connection structure clamping, magnetic connection and so on. In some embodiments, in the ultrasonic surgical tool 100, the jaw body 152 of the jaw assembly (e.g., as shown in FIG. 4A, FIG. 4B) and the distal section 1112 of the ultrasonic blade may form a bipolar electrosurgical tool to perform a coagulation operation on the biological tissue when the jaw body 152 and the distal section 1112 of the ultrasonic blade sandwich the biological tissue.

FIG. 4A shows a structural schematic diagram of another part of the structure of an ultrasonic surgical tool 100 according to some embodiments of the present disclosure. FIG. 4B shows a cross-sectional schematic diagram of another part of the structure of an ultrasonic surgical tool 100 according to some embodiments of the present disclosure. FIG. 5 shows a structural schematic diagram of an ultrasonic surgical tool 100 according to some embodiments of the present disclosure.

In some embodiments, the ultrasonic blade 111 may also comprise a flange 1113. As shown in FIG. 1A, FIG. 1B and FIG. 4B, the flange 1113 may be disposed between the proximal section 1111 and the distal section 1112 of the ultrasonic blade. In some embodiments, the proximal section 1111, the distal section 1112, and the flange 1113 of the ultrasonic blade assembly 110 may be integrally formed.

In some embodiments, as shown in FIG. 4A or FIG. 4B, the ultrasonic blade assembly 110 may also comprise an ultrasonic blade shell 112. As shown in FIG. 4B, the ultrasonic blade shell 112 may cover at least a portion of the proximal section 1111 and distal section 1112 of the ultrasonic blade 111 and engages with the flange 1113. The distal section 1112 of the ultrasonic blade 111 extends distally out of the ultrasonic blade shell 112, and the distal end of the distal section 1112 forms an ultrasonic blade head. In an operation, the distal end of the distal section 1112 cuts or coagulates the biological tissue as it comes into contact with the biological tissue.

In some embodiments, as shown in FIG. 4B, the ultrasonic blade shell 112 may comprise a proximal ultrasonic blade shell 1121 and a distal ultrasonic blade shell 1122. The proximal ultrasonic blade shell 1121 covers at least a portion of the proximal section 1111 of the ultrasonic blade 111, and the distal ultrasonic blade shell 1122 covers at least a portion of the distal section 1112 of the ultrasonic blade 111. In some embodiments, the proximal ultrasonic blade shell 1121 and the distal ultrasonic blade shell 1122 are detachably connected. In some embodiments, a connection part of the proximal ultrasonic blade shell 1121 and the distal ultrasonic blade shell 1122 (e.g., the connection part indicated by Z in FIG. 4A) is engaged with the flange 1113.

In some embodiments, as shown in FIG. 4B, the proximal ultrasonic blade shell 1121 may comprise a first step 11211 disposed at the distal end of the proximal ultrasonic blade shell 1121 for being propped against the proximal end of the flange 1113. At the surface 403 formed by the first step 11211, the proximal ultrasonic blade shell 1121 may be connected to the flange 1113 in a suitable manner such as a threaded connection, or may be simply abutted. As shown in FIG. 4B, the proximal ultrasonic blade shell 1121 may also comprise a second step 11212 disposed at the distal end of the proximal ultrasonic blade shell 1121 for being propped against the proximal end of the distal ultrasonic blade shell 1122. At the surface 404 formed by the second step 11212, the proximal ultrasonic blade shell 1121 may be connected in a suitable manner to the inner surface of the proximal end of the distal ultrasonic blade shell 1122, such as a threaded connection or the like.

In some embodiments, when mounting the ultrasonic blade shell 112, the proximal ultrasonic blade shell 1121 can be sleeved on the proximal section 1111 from the proximal end of the ultrasonic blade 111, so that the first step 11211 at the distal end of the proximal ultrasonic blade shell 1121 is in contact with the proximal end of the flange 1113. Further, the proximal ultrasonic blade shell 1121 is connected to the flange 1113 at 403 by means of threaded connection, abutting connection or the like. Further, the distal ultrasonic blade shell 1122 is sleeved on the distal section 1112 by the distal end of the ultrasonic blade 111.The distal ultrasonic blade shell 1122 and proximal ultrasonic blade shell 1121 are connected in a manner of threaded connection or the like at 404. The proximal end of the distal ultrasonic blade shell 1122 is propped against the second step 11212 of the distal end of the proximal ultrasonic blade shell 1121, at which time the proximal end of the distal ultrasonic blade shell 1122 can be propped against the distal end of the flange 1113 (e.g., at point T shown in FIG. 4B). Therefore, the connection part of the distal ultrasonic blade shell 1122 and proximal ultrasonic blade shell 1121 is engaged with the flange 1113.

As shown in FIG. 5, in the ultrasonic surgical tool 100, the ultrasonic blade assembly 110 may be disposed at the distal end of the ultrasonic transducer 120. In some embodiments, as shown in FIG. 5, the diameter of the distal end of the ultrasonic blade shell 112 and the diameter of the proximal end of the ultrasonic transducer shell 125 may be equal.

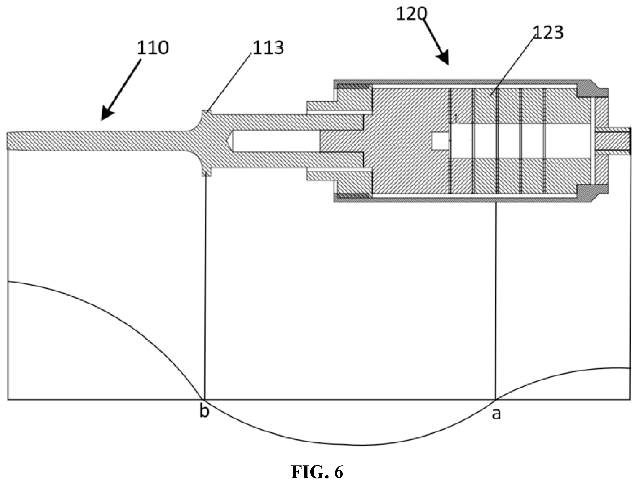

FIG. 6 shows an amplitude distribution of an ultrasonic surgical tool 100 according to some embodiments of the present disclosure. It may be understood by those skilled in the art that the amplitude node refers to a position where the amplitude is zero when the wave propagates in the medium. For the ultrasonic surgical tool 100, the amplitude node refers to a position where the amplitude of the mechanical wave is zero as it propagates in the ultrasonic surgical tool 100. In FIG. 6, the amplitude node corresponds to the position of the zero point on the amplitude distribution curve, such as the first amplitude node a and the second amplitude node b in FIG. 6. In some embodiments, as shown in FIG. 6, the first amplitude node a of the ultrasonic surgical tool 100 may be located in the ultrasonic transducer section 123 of the ultrasonic transducer 120. It may be understood by those skilled in the art that the greater the amplitude, the greater the energy loss caused by damping. The ultrasonic transducer section is the part with large mechanical vibration damping in the ultrasonic surgical tool. For the ultrasonic surgical tool 100, the first amplitude node a is set in the ultrasonic transducer section 123, which can reduce the energy loss of the ultrasonic transducer section 123, so as to improve the energy transmitted to the ultrasonic blade 111, so as to improve the energy of the distal end of the ultrasonic blade 111. This improves the efficiency of the surgical operations. In some embodiments, the first amplitude node a of the ultrasonic surgical tool 100 may be located at the midpoint of the ultrasonic transducer section 123 of the ultrasonic transducer 120 to minimize the amplitude of the ultrasonic transducer section 123 and the corresponding energy loss. It helps to maximize the output power of the ultrasonic transducer 120.

As shown in FIG. 6, in some embodiments, the ultrasonic surgical tool 100 further comprises a second amplitude node b. In some embodiments, the second amplitude node b of the ultrasonic surgical tool 100 is located at the flange 1113 of the ultrasonic blade 111. The flange 1113 is connected with the ultrasonic blade shell (not shown in FIG. 6, as the ultrasonic blade shell 112 shown in FIG. 4B). The second amplitude node b of the ultrasonic surgical tool 100 is set at the flange 1113 of the ultrasonic blade 111, which can avoid the vibration of the flange 1113. Thus, the vibration of the ultrasonic blade shell (not shown in FIG. 6, such as the ultrasonic blade shell 112 shown in FIG. 4B) can be prevented, so that the stability of the surgical tool can be improved, and the safety of the operation can be improved.

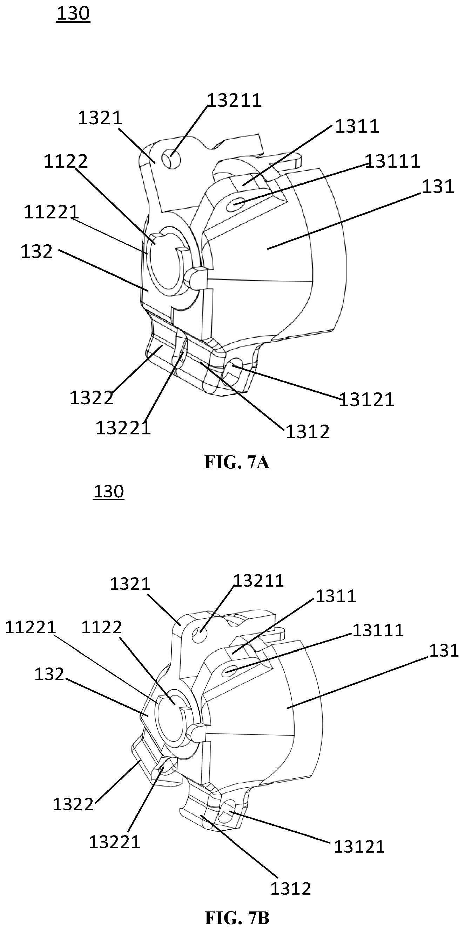

As shown in FIG. 4A or FIG. 5, the clamping assembly 130 may be connected with the ultrasonic blade assembly 110, and the clamping assembly 130 may be configured to mount or demount the ultrasonic surgical tool 100. FIG. 7A shows a structural schematic diagram of a clamping assembly 130 according to some embodiments of the present disclosure. FIG. 7B shows a structural schematic diagram of a clamping assembly 130 according to some embodiments of the present disclosure in another state. In some embodiments, the clamping assembly 130 may be a clamp structure, as shown in FIG. 7A or FIG. 7B.The clamping assembly 130 may comprise a fixed clamp 131 and a movable clamp 132. The fixed clamp 131 may be fixedly connected with the distal ultrasonic blade shell 1122, for example, by welding or the like. The movable clamp 132 may be rotatably connected with the distal ultrasonic blade shell 1122 and cooperate with the fixed clamp 131.

In some embodiments, as shown in FIG. 7A or FIG. 7B, the fixed clamp 131 comprises an upper fixed clamping ear 1311 and a lower fixed clamping ear 1312. The movable clamp 132 comprises an upper movable clamping ear 1321 and a lower movable clamping ear 1322. The upper fixed clamping ear 1311 cooperates with the upper movable clamping ear 1321, and the lower fixed clamping ear 1312 cooperates with the lower movable clamping ear 1322. In some embodiments, the upper fixed clamping ear 1311 and the upper movable clamping ear 1321 tend to be closed, for example, the upper fixed clamping ear 1311 and the upper movable clamping ear 1321 may move from a relative position as shown in FIG. 7A to a relative position as shown in FIG. 7B. At this time, the lower fixed clamping ear 1312 and the lower movable clamping ear 1322 tend to open, for example, the lower fixed clamping ear 1312 and the lower movable clamping ear 1322 may move from a relative position as shown in FIG. 7A to a relative position as shown in FIG. 7B. It may be understood by those skilled in the art that FIG. 7B shows a state which the upper fixed clamping ear 1311 and the upper movable clamping ear 1321 may reach during the closing process, and in some cases, the upper fixed clamping ear 1311 and the upper movable clamping ear 1321 can be closed to fully fit.

In some embodiments, the upper fixed clamping ear 1311 and the upper movable clamping ear 1321 tend to open. For example, the upper fixed clamping ear 1311 and the upper movable clamping ear 1321 may move from the relative position as shown in FIG. 7B to the relative position as shown in FIG. 7A. At this time, the lower fixed clamping ear 1312 and the lower movable clamping ear 1322 tend to be closed.For example, the lower fixed clamping ear 1312 and the lower movable clamping ear 1322 may move from the relative position shown in FIG. 7B to the relative position shown in FIG. 7A.

In some embodiments, as shown in FIG. 4A, FIG. 4B, or FIG. 5, the ultrasonic surgical tool 100 may also comprise a clamping torsional spring 140. As shown in FIG. 4A, FIG. 4B, or FIG. 5, at least a portion of the clamping torsional spring 140 may be sleeved on the ultrasonic blade shell 112. A distal end of the clamping torsional spring 140 may be connected with the movable clamp 132 of the clamping assembly 130 (e.g., at a point O as shown in FIG. 4B, fixedly connected by welding or the like or in abutment). The proximal end of the clamping torsional spring 140 may be fixedly connected with the proximal ultrasonic blade shell 1121 (e.g., the proximal end of the clamping torsional spring 140 is fixed in a hole comprised in the proximal ultrasonic blade shell 1121). The clamping torsional spring 140 may be configured to apply a torque to the upper movable clamping ear 1321 to make the upper movable clamping ear 1321 tend away from the upper fixed clamping ear 1311, thereby causing the lower fixed clamping ear 1312 and the lower movable clamping ear 1322 to tend to be closed. It may be understood by those skilled in the art that, based on the torque applied by the clamping torsional spring 140 to the upper movable clamping ear 1321 of the clamping assembly 130, in a natural state, the upper fixed clamping ear 1311 and the upper movable clamping ear 1321 are opened and the lower fixed clamping ear 1312 and the lower movable clamping ear 1322 are closed.

In some embodiments, the lower fixed clamping ear 1312 may comprise a dent or a hole for mounting (e.g., a through hole 13121 provided on the lower fixed clamping ear 1312 shown in FIG. 7A or FIG. 7B). The lower movable clamping ear 1322 may comprise a dent or a hole for mounting (e.g., a through hole 13221 provided on the lower movable clamping ear 1322 shown in FIG. 7A or FIG. 7B). In some embodiments, the upper fixed clamping ear 1311 may comprise a dent or a hole for engaging with a clamp applicator (not shown) (e.g., a through hole 13111 provided on the upper fixed clamping ear 1311 shown in FIG. 7A or FIG. 7B).The upper movable clamping ear 1321 may comprise a dent or a hole for engaging with the clamp applicator (e.g., a through hole 13211 provided on the upper movable clamping ear 1321 shown in FIG. 7A or FIG. 7B).

Based on the dent or hole provided on the upper fixed clamping ear 1311 and the dent or hole provided on the upper movable clamping ear 1321, the upper fixed clamping ear 1311 and the upper movable clamping ear 1321 can be clamped by the clamp applicator.Therefore, the upper fixed clamping ear 1311 and the upper movable clamping ear 1321 are closed, and the lower fixed clamping ear 1312 and the lower movable clamping ear 1322 are opened. Under the condition that the lower fixed clamping ear 1312 and the lower movable clamping ear 1322 are opened, it is convenient for assembling operations.

In some embodiments, as shown in FIG. 4A, FIG. 4B, or FIG. 5, the ultrasonic surgical tool 100 may also comprise a jaw assembly 150. As shown in FIG. 4A, FIG. 4B, or FIG. 5, the jaw assembly 150 may be disposed at a distal end of the clamping assembly 130. The jaw assembly 150 may comprise a jaw base 151 and a jaw body 152. The jaw base 151 may be connected to the distal end of the clamping assembly 130. In some embodiments, the jaw base 151 may also be fixedly connected to the distal ultrasonic blade shell 1122, for example, the jaw base 151 may be welded to the distal end surface 11221 of the distal ultrasonic blade shell 1122 (as shown in FIG. 7A, FIG. 7B). The jaw body 152 may be disposed at a distal end of the jaw base 151, and the proximal end of the jaw body 152 may be hinged to the jaw base 151 (e.g., hinged with the jaw base 151 at a point Q as shown in FIG. 4A and a point P as shown in FIG. 4B). It may be understood by those skilled in the art that the point Q and the point P are oppositely disposed on the jaw base 151. The oppositely disposed to each other means that the longitudinal section required for obtaining the sectional view as shown in FIG. 4B is a symmetrical plane, which is symmetrically disposed.

In some embodiments, the jaw base 151 may also comprise a first sliding groove 1511 as shown in FIG. 4A and a second sliding groove 1512 as shown in FIG. 4B. As shown in FIG. 4A and FIG. 4B, the first sliding groove 1511 may be disposed on a first side of the jaw base 151, and the second sliding groove 1512 may be disposed on a second side of the jaw base 151, and the first sliding groove 1511 and the second sliding groove 1512 are oppositely disposed. The jaw assembly 150 may also comprise at least one connecting rod (e.g., the first connecting rod 153 shown in Figure 4A and/or the second connecting rod 154 shown in Figure 4B), a distal end of the at least one connecting rod being hinged to the proximal end of the jaw body 152. For example, the distal end of the first connecting rod 153 is hinged to the proximal end of the jaw body 152 at point R as shown in FIG. 4A; the distal end of the second connecting rod 154 is hinged to the proximal end of the jaw body 152 at point S as shown in Figure 4B.

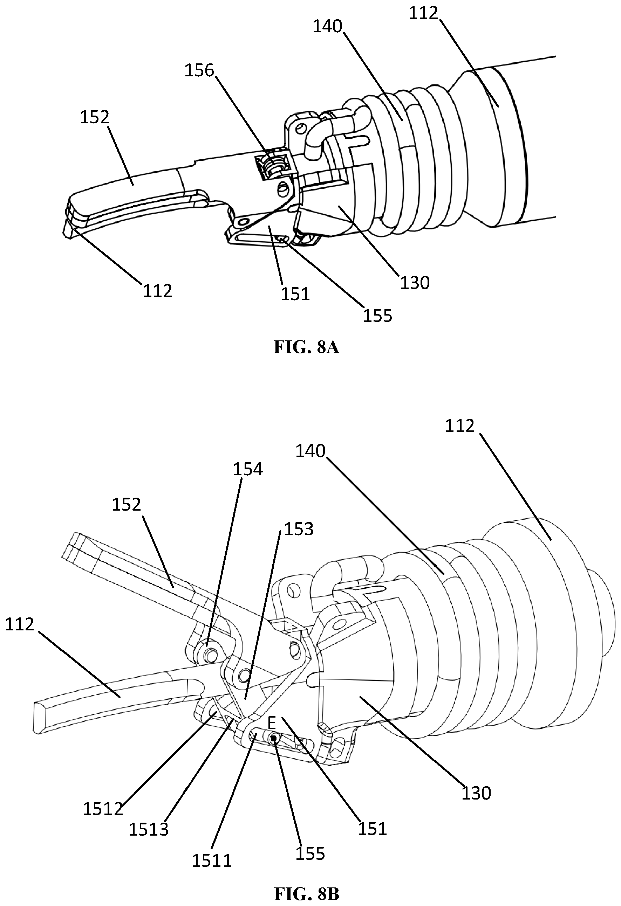

FIG. 8A shows a structural schematic diagram of yet another part of the structure of an ultrasonic surgical tool 100 according to some embodiments of the present disclosure. FIG. 8B shows a structural schematic diagram of yet another part of the structure of an ultrasonic surgical tool 100 according to some embodiments of the present disclosure at another angle. As shown in FIG. 4A, FIG. 4B, or FIG. 8B, the jaw assembly 150 may also comprise a pin structure 155. The pin structure 155 may be hinged to a proximal end of the at least one connecting rod, for example, to the first connecting rod 153 at a position as indicated by point C in Figure 4A and/or to the second connecting rod 154 at a position as indicated by point D in Figure 4B. Two ends of the pin structure 155 may be slidingly connected with the first sliding groove 1511 and the second sliding groove 1512, respectively, for example, one end of the pin structure 155 may be slidingly connected with the first sliding groove 1511 at a position indicated by E in FIG. 8B. The other end of the pin structure 155 may be slidingly connected with the second sliding groove 1512 at a position corresponding to the position indicated by E.

In some embodiments, as shown in FIG. 8B, at least one connecting rod may comprise a first connecting rod 153 and a second connecting rod 154. The first connecting rod 153 and the second connecting rod 154 are connected to form an H-shaped link (e.g., an H-shaped link by a cross rod 1513 as shown in FIG. 8B). As shown in FIG. 8B, the ultrasonic blade 111 may extend through the jaw base 151 and the H-shaped link (the H-shaped link consisting of the first connecting rod 153, the second connecting rod 154 and the cross rod 1513). As shown in FIG. 8B, the axis of the pin structure 155 may be parallel to the cross rod 1513.

In some embodiments, the opening and closing of a jaw formed by the ultrasonic blade 111 and the jaw body 152 can be controlled by moving the pin structure 155. For example, the pin structure 155 can be pushed in the direction indicated by the arrow 401 in FIG. 4A, so that both ends of the pin structure 155 may slide in the direction indicated by the arrow 401 in the first sliding groove 1511 and the second sliding groove 1512. Under the driving of the pin structure 155, the first connecting rod 153 and the second connecting rod 154 may move upwards. Pushed by the first connecting rod 153 and the second connecting rod 154, the jaw body 152 takes the straight line where the point Q shown in FIG. 4A and the point P shown in FIG. 4B lie as the rotating shaft and rotates towards the direction indicated by the arrow 402 in FIG. 4A. The jaw body 152 tends to be far away from the ultrasonic blade 111. Thus, the jaw formed by the ultrasonic blade 111 and the jaw body 152 may be opened.

In an operation, the jaw formed by the ultrasonic blade 111 and the jaw body 152 can be used for clamping the biological tissue by controlling the jaw body 152 to be closed, so that the ultrasonic blade assembly 110 can perform tissue cutting to the biological tissue while clamping the biological tissue, which is convenient for the tissue cutting.

In some embodiments, as shown in FIG. 8A, the jaw assembly 150 may also comprise a jaw torsional spring 156. The jaw torsional spring 156 may be disposed between the jaw base 151 and the jaw body 152. The jaw torsional spring 156 is configured to apply a torque to the jaw body 152 to make the jaw body 152 tend to be closed (e.g., to make the jaw body 152 tend to approach the distal section 1112 of the ultrasonic blade). It may be understood by those skilled in the art that based on the jaw torsional spring 156, in a natural state, the jaw body 152 remains in the closed state as shown in Figure 8A, and the jaw body 152 is in contact with the distal section 1112 of the ultrasonic blade. Based on the jaw torsional spring 156, when no surgical operation is performed, the jaw body 152 remains closed, and the two ends of the pin structure 155 are located at the nearest ends of the first sliding groove 1511 and the second sliding groove 1512 as shown in FIG. 8A, thereby facilitating the entry and exit of the ultrasonic surgical tool 100 into and out of the patient's body.

In some embodiments, as shown in FIG. 4A or FIG. 4B, the jaw assembly 150 may also comprise a jaw pad 157. The jaw pad 157 may be disposed on the jaw body 152. The jaw pad 157 can be connected to the jaw body 152 through a T-shaped groove. In some embodiments, the jaw pad 157 may be made of PTFE (polytetrafluoroethylene), PEEK (polyetheretherketone) and other soft materials to avoid damage to the ultrasonic blade 111 that vibrates at high speed in the event that the jaw body 152 is fully closed (in close contact with the distal section 1112 of the ultrasonic blade 111).

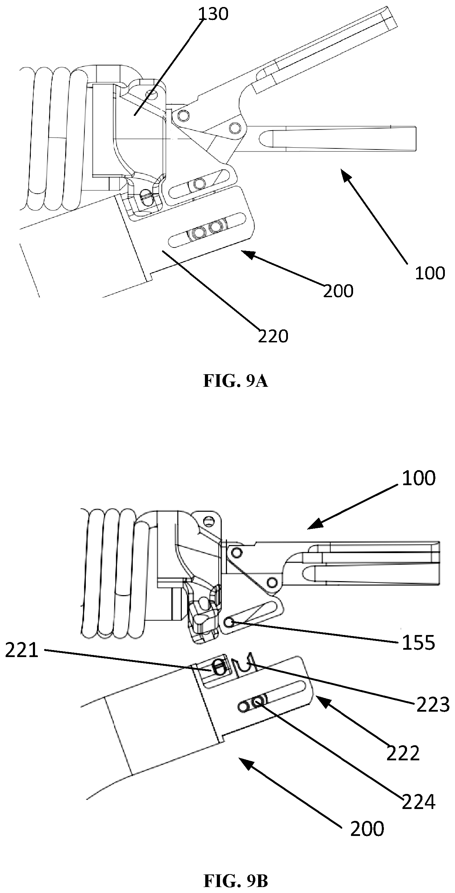

Some embodiments of the present disclosure also provide an assemblable ultrasonic surgical tool assembly. The assemblable ultrasonic surgical tool assembly may comprise an ultrasonic surgical tool 100 as in any of some embodiments of the present disclosure and an auxiliary surgical instrument 200. FIG. 9A shows a schematic diagram of the connection relationship between an ultrasonic surgical tool 100 and an auxiliary surgical instrument 200 according to some embodiments of the present disclosure. FIG. 9B shows a structural schematic diagram of part of the structure of an ultrasonic surgical tool 100 and an auxiliary surgical instrument 200 according to some embodiments of the present disclosure. As shown in FIGS. 9A and 9B, the auxiliary surgical instrument 200 may be detachably connected with the ultrasonic surgical tool 100. In FIG. 9A, the ultrasonic surgical tool 100 and the auxiliary surgical instrument 200 are in a connected state; in FIG. 9B, the ultrasonic surgical tool 100 and the auxiliary surgical instrument 200 are in a state of separation or awaiting connection. The auxiliary surgical instrument 200 may be configured to drive the ultrasonic surgical tool 100 to move. As shown in FIG. 2, the auxiliary surgical instrument 200 may comprise an instrument stem 210 (only a small portion of the distal end of the instrument stem 210 is shown in FIG. 2) and an instrument end effector 220 disposed at a distal end of the instrument stem 210. As shown in FIG. 9A or FIG. 9B, the instrument end effector 220 can be detachably connected with the clamping assembly 130 of the ultrasonic surgical tool 100.

In some embodiments, as shown in FIG. 2, the instrument end effector 220 of the auxiliary surgical instrument 200 comprises a connecting part 221. As shown in FIG. 2, the connecting part 221 may comprise a connecting column 2211 extending toward the distal end of the instrument stem, and a first bump structure 2212 and a second bump structure 2213 disposed on two sides of the connecting column 2211. The first bump structure 2212 and the second bump structure 2213 may be configured to connect with the ultrasonic surgical tool 100.

In some embodiments, in the ultrasonic surgical tool 100, the lower fixed clamping ear 1312 of the clamping assembly 130 may comprise a dent or a hole for mounting (e.g., a through hole 13121 provided on the lower fixed clamping ear 1312 shown in FIG. 7A or FIG. 7B), and the dent or hole may be configured to receive at least a portion of the first bump structure 2212 of the auxiliary surgical instrument 200. The lower movable clamping ear 1322 of the clamping assembly 130 may comprise a dent or a hole for mounting (e.g., a through hole 13221 provided on the lower movable clamping ear 1322 shown in FIG. 7A or FIG. 7B), and the dent or hole may be configured to receive the second bump structure 2213 of the auxiliary surgical instrument 200. Under the condition that the dent or hole of the lower fixed clamping ear 1312 of the ultrasonic surgical tool 100 is clamped with the first bump structure 2212 of the auxiliary surgical instrument 200 and the dent or hole of the lower movable clamping ear 1322 is clamped with the second bump structure 2213, the ultrasonic surgical tool 100 and the auxiliary surgical instrument 200 can form a rigid connection. As a result, it is convenient to control the movement of the auxiliary surgical instrument 200 to drive the movement of the ultrasonic surgical tool 100.

In some embodiments, the proximal end of the auxiliary surgical instrument 200 may be configured to connect with a driving device for providing a driving force for the movement of the auxiliary surgical instrument 200 (e.g., movement in a patient's body). The driving device can drive the auxiliary surgical instrument 200 to move in a patient's body. The auxiliary surgical instrument 200 may drive the ultrasonic surgical tool 100 to move in the patient's body.

It may be understood by those skilled in the art that the construction of a rigid connection between the ultrasonic surgical tool 100 and the auxiliary surgical instrument 200 is not limited to the structures described above (the connecting part 221 of the auxiliary surgical instrument 200, and a dent or a hole of the lower fixed clamping ear 1312 and the lower movable clamping ear 1322 of the ultrasonic surgical tool 100 matched with it). It is also possible to establish a rigid connection between the ultrasonic surgical tool 100 and the auxiliary surgical instrument 200 by any suitable structure such as the clamping structure and magnetic attraction structure, which are respectively disposed on the ultrasonic surgical tool 100 and the auxiliary surgical instrument 200 and cooperate with each other.

In some embodiments, as shown in FIG. 2, the instrument end effector 220 may also comprise a body 222, which may comprise a sliding space 222c and the third sliding groove 222a and the fourth sliding groove 222b located at two opposite sides of the sliding space 222c. As shown in FIG. 2, the instrument end effector 220 may also comprise a sliding block 223, which may be slidingly connected with the third sliding groove 222a and fourth sliding groove 222b by at least one pin (e.g., two pins indicated by 224 shown in FIG. 2) or bump. The sliding block 223 may comprise at least one groove structure (e.g., groove structure 2231 shown in FIG. 2). At least one groove structure 2231 may be provided on an upper part of the sliding block 223 and disposed above the sliding space 222c, and the at least one groove structure 2231 may be configured to connect with the ultrasonic surgical tool 100. It may be understood by those skilled in the art that the sliding block 223 may move in the sliding space 222c when at least one pin 224 or bump slides in the third sliding groove 222a and fourth sliding groove 222b. In some embodiments, the groove structure 2231 may be a U-shaped groove.

In some embodiments, as shown in FIG. 2, the body 222 may comprise a first arm 2221 and a second arm 2222. The first arm 2221 and the second arm 2222 may be oppositely disposed on two sides of the sliding space 222c and extend toward the distal end of the instrument stem 210. As shown in FIG. 2, the first arm 2221 may comprise a third sliding groove 222a, and the second arm 2222 may comprise a fourth sliding groove 222b.

In some embodiments, as shown in FIG. 4A or FIG. 4B, the ultrasonic surgical tool 100 may comprise a jaw assembly 150, which may comprise a jaw base 151, and a jaw body 152,at least one connecting rod (e.g., a first connecting rod 153 and a second connecting rod 154) and a pin structure 155. At least one groove structure 2231 comprised in the sliding block 223 can be engaged with the pin structure 155.

In some embodiments, in the case that the groove structure 2231 of the sliding block 223 is engaged with the pin structure 155, the sliding block 223 may move in the sliding space 222c and may drive the pin structure 155 to move (the two ends of the pin structure 155 may slide in the first sliding groove 1511 and the second sliding groove 1512). The movement of the pin structure 155 may drive the movement of the first connecting rod 153 and the second connecting rod 154, and may push the opening and closing of the jaw body 152.

In some embodiments, the auxiliary surgical instrument 200 may also comprise a driving wire (not shown). The driving wire may pass through the instrument stem 210 of the auxiliary surgical instrument 200 (e.g., through the central through hole of the instrument stem 210). A distal end of the driving wire may be connected with the sliding block 223, and the driving wire may be configured to drive the sliding block 223 to slide along the third sliding groove 222a and the fourth sliding groove 222b. In some embodiments, a proximal end of the driving wire may pass through the instrument stem 210 to connect with a sliding block driving device configured to provide a driving force for the movement of the sliding block 223. The sliding block driving device may push and pull the driving wire, so that the sliding block 223 may move in the sliding space 222c, and may further drive the opening and closing of the jaw body 152 of the ultrasonic surgical tool 100. In some embodiments, the driving wire may be a nickel-titanium alloy wire.

In some embodiments, the assemblable ultrasonic surgical tool assembly provided by the present disclosure may also comprise a clamp applicator (not shown). The clamp applicator may comprise a first clamp body and a second clamp body. A distal end of the first clamp body is provided with a third bump structure, and a distal end of the second clamp body is provided with a fourth bump structure. The clamp applicator may be configured to clamp the upper fixed clamping ear 1311 and the upper movable clamping ear 1321 of the clamping assembly 130, so that the upper fixed clamping ear 1311 and the upper movable clamping ear 1321 may be closed, and the lower fixed clamping ear 1312 and the lower movable clamping ear 1322 of the clamping assembly 130 may be opened. The assembly of the ultrasonic surgical tool 100 and the auxiliary surgical instrument 200 is facilitated.

In some embodiments, in the ultrasonic surgical tool 100, the upper fixed clamping ear 1311 may comprise a dent or a hole (e.g., a through hole 13111 disposed on the upper fixed clamping ear 1311 shown in FIG. 7A or FIG. 7B) for receiving the third bump structure. The upper movable clamping ear 1321 may comprise a dent or a hole (e.g., a through hole 13211 disposed on the upper movable clamping ear 1321 shown in FIG. 7A or FIG. 7B) for receiving the fourth bump structure. Based on the third bump structure and the fourth bump structure of the clamp applicator and the dent or hole of the upper fixed clamping ear 1311 and the upper movable clamping ear 1321 of the ultrasonic surgical tool 100, the stability of the clamp applicator in clamping the upper fixed clamping ear 1311 and the upper movable clamping ear 1321 of the clamping assembly 130 may be enhanced.

It may be understood by those skilled in the art that the connecting structure enabling the clamp applicator to clamp the upper fixed clamping ear 1311 and the upper movable clamping ear 1321 of the ultrasonic surgical tool 100 is not limited to the above structures (including the third bump structure and the fourth bump structure of the clamp applicator and the dent or hole of the upper fixed clamping ear 1311 and the upper movable clamping ear 1321). It may also be any other suitable structure, for example, connecting structures or the like that can be engaged with each other, and that are respectively provided on the first clamp body, the second clamp body, the upper fixed clamping ear 1311 and the upper movable clamping ear 1321. In some embodiments, a non-slip material may also be disposed on the first clamp body and the second clamp body of the clamp applicator and on the upper fixed clamping ear 1311 and the upper movable clamping ear 1321, whereby the ultrasonic surgical tool 100 may be directly clamped by the clamp applicator.

In some embodiments, prior to performing the surgical operation by the assemblable ultrasonic surgical tool assembly, the ultrasonic surgical tool 100 and the auxiliary surgical instrument 200 may be assembled by the following process (including steps 1 to 6) :
Step 1, place the distal end of the ultrasonic surgical tool 100 into a patient's body.

In some embodiments, the ultrasonic surgical tool 100 may also comprise a traction rope (not shown) disposed at a proximal end of the ultrasonic transducer 120, a distal end of the traction rope being connected with the proximal end of the ultrasonic transducer 120, a proximal end of the traction rope being held by a user (e.g., a doctor or a nurse). In step 1, the distal end of the ultrasonic surgical tool 100 may be placed into a patient's body through a traction rope. In some embodiments, the wire bundle comprised by the plurality of wires of the ultrasonic transducer 120 may comprise an insulating trocar. Based on this, in step 1, the wire bundle can be pulled and the distal end of the ultrasonic surgical tool 100 can be placed into the patient's body through the wire bundle. In some embodiments, during placement of the distal end of the ultrasonic surgical tool 100 into the patient's body, the jaw body 152 of the ultrasonic surgical tool 100 remains closed to facilitate access into the patient's body and avoid touching tissue of the patient.

Step 2, extend the distal end of the auxiliary surgical instrument 200 into the patient's body.

In step 2, the distal end of the trocar may be first extended into the patient's body, and in turn the distal end of the auxiliary surgical instrument 200 may be to extended into the patient's body through a channel provided by the trocar. It may be understood by those skilled in the art that the trocar may be configured to provide channels into a patient's body for surgical instruments. In some embodiments, the trocar may allow a plurality of surgical instruments to enter the patient's body through the trocar. In some embodiments, the auxiliary surgical instrument 200 is disposed at the distal end of a robotic arm of a surgical robot system (e.g., the robotic arm 311 of the surgical robot system 300). In step 2, the surgical robot system (e.g., a driving device of the surgical robot system for driving the auxiliary surgical instrument 200) may control the distal end of the auxiliary surgical instrument 200 to extend into the patient's body through the trocar.

step 3, extend the distal end of the clamp applicator into the patient's body.

In step 3, the clamp applicator can be extended into the patient's body through the channel provided by the trocar. In some embodiments, the clamp applicator may be held by a user and the distal end of the clamp applicator may be placed into the patient's body through the trocar. In some embodiments, the clamp applicator may be disposed at the distal end of the robotic arm of the surgical robot system (e.g., the robotic arm 311 of the surgical robot system 300). In step 3, the surgical robot system (e.g., a driving device of the surgical robot system for driving the clamp applicator) may control the distal end of the clamp applicator extend into the patient's body through the trocar.

Step 4, use the clamp applicator to clamp the upper fixed clamping ear 1311 and the upper movable clamping ear 1321 of the clamping assembly 130 of the ultrasonic surgical tool 100.

In some embodiments, in a natural state, the upper fixed clamping ear 1311 and the upper movable clamping ear 1321 of the clamping assembly 130 may open, and the lower fixed clamping ear 1312 and the lower movable clamping ear 1322 may be closed. Based on the step 4, the upper fixed clamping ear 1311 and the upper movable clamping ear 1321 may be closed, so that the lower fixed clamping ear 1312 and the lower movable clamping ear 1322 may be opened, and the ultrasonic surgical tool 100 may be in a state convenient for assembling with the auxiliary surgical instrument 200. In some embodiments, the first clamp body and the second clamp body of the clamp applicator respectively comprise a third bump structure and a fourth bump structure, and the upper fixed clamping ear 1311 and the upper movable clamping ear 1321 of the ultrasonic surgical tool 100 respectively comprise a dent or a hole. In step 4, the third bump structure may engage with the hole 13111 on the upper fixed clamping ear 1311, the fourth bump structure may engage with the hole 13211 on the upper movable clamping ear 1321, so that the clamp applicator may be stably connected with the ultrasonic surgical tool 100, so as to clamp the upper fixed clamping ear 1311 and the upper movable clamping ear 1321 of the ultrasonic surgical tool 100 by the clamp applicator.

In some embodiments, in step 4, the clamp applicator may be held by a user and used to clamp the upper fixed clamping ear 1311 and the upper movable clamping ear 1321 of the clamping assembly 130 of the ultrasonic surgical tool 100. In some embodiments, the clamp applicator may be disposed at a distal end of a robotic arm of a surgical robot system (e.g., the robotic arm 311 of the surgical robot system 300). In step 4, the surgical robot system (e.g., a driving device of the surgical robot system for driving the clamp applicator to perform surgical operations) may control the clamp applicator to clamp the upper fixed clamping ear 1311 and the upper movable clamping ear 1321 of the clamping assembly 130 of the ultrasonic surgical tool 100.

Step 5, adjust the positions of the ultrasonic surgical tool 100 and the auxiliary surgical instrument 200 in the patient's body to positions convenient for assembly.

In step 5, the ultrasonic surgical tool 100 and the auxiliary surgical instrument 200 may be moved to a position as shown in FIG. 9B. In some embodiments, the ultrasonic surgical tool 100 may be adjusted to a position convenient for assembly by a user (e.g., a doctor, a nurse, or the like) through a traction rope or wire bundle. Since the clamp applicator and the ultrasonic surgical tool 100 establish a rigid connection through step 4, the ultrasonic surgical tool 100 can also be adjusted to a position convenient for assembly by the clamp applicator. In some embodiments, the auxiliary surgical instrument 200 may be controlled to move to a position near the ultrasonic surgical tool 100 and convenient for assembly with the ultrasonic surgical tool 100 by controlling a driving device for driving the auxiliary surgical instrument 200 to move.

Step 6, assemble the ultrasonic surgical tool 100 and the auxiliary surgical instrument 200.

During the assembling process, the jaw body 152 of the ultrasonic surgical tool 100 may remain in a closed state so as to facilitate entry into the patient's body and avoid collision with the tissue of the patient's body. At this time, the two ends of the pin structure 155 are located at the nearest ends of the first sliding groove 1511 and the second sliding groove 1512, as shown in FIG. 9B. Based on this, when performing step 6, at least one pin or bump at the lower part of the sliding block 223 can be located at the nearest end of the third sliding groove 222a and the fourth sliding groove 222b, as shown in FIG. 9B. It is convenient to connect the sliding block 223 of the auxiliary surgical instrument 200 to the pin structure 155 of the ultrasonic surgical tool 100. For example, the sliding block 223 can be moved to the position by pulling the driving wire.

Through step 4, the lower fixed clamping ear 1312 and the lower movable clamping ear 1322 may be opened. Through step 5, the ultrasonic surgical tool 100 and the auxiliary surgical instrument 200 can be moved to a position and pose as shown in FIG. 9B. On the basis of the position and pose as shown in FIG. 9B, the auxiliary surgical instrument 200 can continue to move upward or the ultrasonic surgical tool 100 can continue to move downward, until the groove structure 2231 of the sliding block 223 of the auxiliary surgical instrument 200 is engaged with the pin structure 155 of the jaw assembly 150 of the ultrasonic surgical tool 100. At this time, the ultrasonic surgical tool 100 and the auxiliary surgical instrument 200 have reached the position and pose suitable for assembly. In some embodiments, the auxiliary surgical instrument 200 may be controlled to move until the groove structure 2231 of the sliding block 223 of the auxiliary surgical instrument 200 is engaged with the pin structure 155 of the jaw assembly 150 of the ultrasonic surgical tool 100, by controlling a driving device configured to drive the auxiliary surgical instrument 200.

After reaching the position and pose suitable for assembly, the clamp applicator may release the upper fixed clamping ear 1311 and the upper movable clamping ear 1321 of the clamping assembly 130 (which may be achieved by the relevant staff or by a driving device). Based on the torque applied by the clamping torsional spring 140 on the upper movable clamping ear 1321, the upper fixed clamping ear 1311 and the upper movable clamping ear 1321 may open, and the lower fixed clamping ear 1312 and the lower movable clamping ear 1322 tend to close, clamping the connecting part 221 of the auxiliary surgical instrument 200. In some embodiments, the through hole 13121 of the lower fixed clamping ear 1312 of the ultrasonic surgical tool 100 may be engaged with the first bump structure 2212 of the auxiliary surgical instrument 200, and the through hole 13221 of the lower movable clamping ear 1322 may be engaged with the second bump structure 2213.

Based on the process described above, the in-vivo assembly of the ultrasonic surgical tool 100 and the auxiliary surgical instrument 200 may be completed. The ultrasonic surgical tool 100 and the auxiliary surgical instrument 200 may establish a rigid connection as shown in FIG. 9A, thereby completing the preparatory work for the assemblable ultrasonic surgical tool assembly. It may be understood by those skilled in the art that the preparatory work for the assemblable ultrasonic surgical tool assembly may comprise steps that are not limited to the steps described above, or not limited to the sequence of steps described above. For example, after the position and pose of the ultrasonic surgical tool 100 and the auxiliary surgical instrument 200 in the patient's body are adjusted to a position and pose convenient for assembly, the distal end of the clamp applicator may be placed in the patient's body, and then the clamp applicator may clamp the upper fixed clamping ear 1311 and the upper movable clamping ear 1321 of the clamping assembly 130 of the ultrasonic surgical tool 100.

In some embodiments, the auxiliary surgical instrument 200 may be carried at the distal end of the robotic arm 311 of the operation trolley 310 of the surgical robot system 300. Based on this, after the ultrasonic surgical tool 100 and the auxiliary surgical instrument 200 form a rigid connection in the patient's body, a user can control the movement of the auxiliary surgical instrument 200 by operating the master manipulator 321 of the surgeon console 320, thereby achieving the movement of the ultrasonic surgical tool 100 within the patient's body. This may facilitate the movement of the ultrasonic blade assembly 110 and the jaw assembly 150 at the distal end of the ultrasonic surgical tool 100 to different positions within the patient's body, allowing for surgical operations on biological tissues at different positions. The user can also control the opening and closing of the jaw assembly 150 of the ultrasonic surgical tool 100 by operating the master manipulator 321 of the surgeon console 320. Thus, the clamp formed by the jaw body 152 and the distal section 1112 of the ultrasonic blade 111 of the ultrasonic surgical tool 100 can be configured to clamp biological tissue.

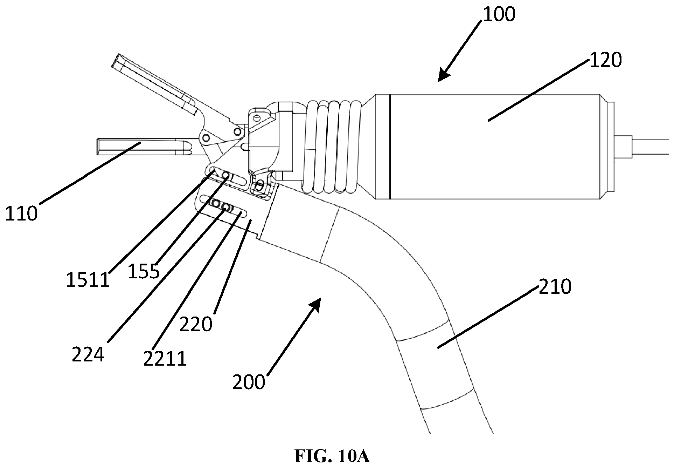

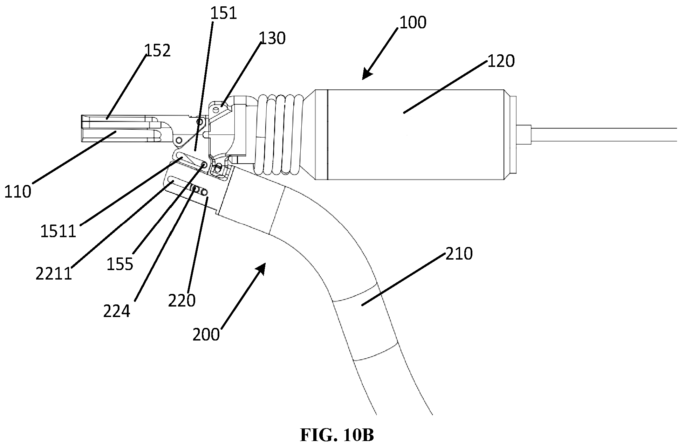

FIG. 10A shows a structural schematic diagram in which the jaw in an assemblable ultrasonic surgical tool assembly according to some embodiments of the present disclosure is in an open state. FIG. 10B shows a structural schematic diagram in which the jaw in an assemblable ultrasonic surgical tool assembly according to some embodiments of the present disclosure is in a closed state. In a natural state, as shown in FIG. 10B, the jaw body 152 may remain closed, and the jaw body 152 may fit against the distal section 1112 of the ultrasonic blade 111, and the two ends of the pin structure 155 may be disposed at the proximal ends of the first sliding groove 1511 and the second sliding groove 1512. Because the groove structure 2231 of the sliding block 223 is engaged with the pin structure 155 of the ultrasonic surgical tool 100, the pin 224 at the lower part of the sliding block 223 may be disposed at the proximal ends of the third sliding groove 222a and the fourth sliding groove 222b, as shown in FIG. 10B.

In some embodiments, under the control of user's operation, the surgical robot system (e.g., the surgical robot system 300 shown in FIG. 3) may push and pull the driving wire connected with the sliding block 223 of the auxiliary surgical instrument 200 so as to push and pull the sliding block 223, to make the two ends of the pin 224 at the lower part of the sliding block 223 to slide forward within the third sliding groove 222a and the fourth sliding groove 222b (slide distally from the proximal ends of the third sliding groove 222a and the fourth sliding groove 222b), for example, slide from the position shown in FIG. 10B to the position shown in FIG. 10A, and to make the sliding block 223 slide distally in a sliding space (e.g., the sliding space 222c shown in FIG. 2), for example, slide from the position shown in FIG. 10B to the position shown in FIG. 10A. Because the groove structure 2231 at the upper part of the sliding block 223 is connected with the pin structure 155 of the ultrasonic surgical tool 100, the forward movement of the sliding block 223 may drive the pin structure 155 to slide distally in the first sliding groove 1511 and the second sliding groove 1512 (slide distally from the proximal ends of the first sliding groove 1511 and the second sliding groove 1512). For example, the pin structure 155 may slide from the position shown in FIG. 10B to the position shown in FIG. 10A. The forward sliding of the pin structure 155 may push the first connecting rod 153 and the second connecting rod 154, and then push the jaw body 152 from the closed state as shown in Figure 10B to the open state as shown in Figure 10A through the first connecting rod 153 and the second connecting rod 154. Based on this, the user can control the opening and closing of the jaw of the ultrasonic surgical tool 100 by operating the master manipulator 321.

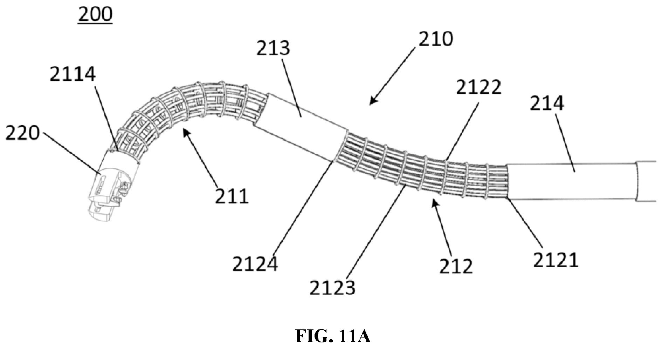

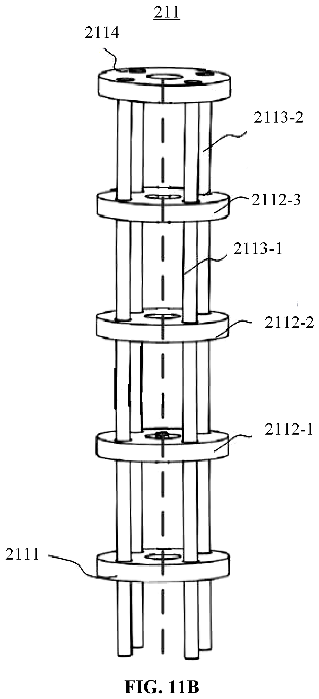

FIG. 11A shows a structural schematic diagram of an auxiliary surgical instrument 200 according to some embodiments of the present disclosure. In some embodiments, the instrument stem 210 may be a flexible arm to increase the degree of freedom of the auxiliary surgical instrument 200 and improve the flexibility of the auxiliary surgical instrument 200 to perform surgical operations in patient's body, and thereby improve the flexibility of the movement of the ultrasonic surgical tool 100 rigidly connected to the auxiliary surgical instrument 200. As shown in FIG. 11A, in some embodiments, the instrument stem 210 of the auxiliary surgical instrument 200 may comprise a first continuum structure 211. FIG. 11B shows a structural schematic diagram of the first continuum structure 211 of an instrument stem 210 according to some embodiments of the present disclosure. As shown in FIG. 11B, the first continuum structure 211 may comprise a first basal disk 2111, a plurality of first spacer disks (e.g., first spacer disks 2112-1, 2112-2, 2112-3 shown in FIG. 11B) and a plurality of first structural backbones (e.g., the first structural backbones 2113-1, 2113-2, etc. shown in FIG. 11B). The plurality of first structural backbones may pass through the plurality of first spacer disks and the first basal disk 2111, and proximal ends of the plurality of first structural backbones may be configured to receive push or pull driving to drive the first continuum structure 211 to move. In some embodiments, as shown in FIG. 11B, the first continuum structure 211 may also comprise a first fixing disk 2114. The distal ends of the plurality of first structural backbones are fixedly connected with the first fixing disk 2114. In some embodiments, as shown in FIG. 11A, the first fixing disk 2114 may be fixedly connected with the proximal end of the instrument end effector 220 of the auxiliary surgical instrument 200.

As shown in FIG. 11B, the plurality of first spacer disks may be disposed at intervals to enhance the stability of a plurality of first structural backbones when being pushed or pulled. The first continuum structure 211 shown in FIG. 11B comprises three first spacer disks. It may be understood by those skilled in the art that the number of the first spacer disks comprised in the first continuum structure 211 is not limited to three. The first continuum structure 211 can comprise any appropriate number of first spacer disks.

In some embodiments, the shapes of the first basal disk 2111, the first spacer disk, and the first fixing disk 2114 may be suitable structures such as an annular structure, a disk-shaped structure, and the like, and the cross section may be various shapes such as a circle, a rectangle, a polygon, and the like.

In some embodiments, as shown in FIG. 11A, the instrument stem 210 may also comprise a second continuum structure 212. The structure of the second continuum structure 212 may be similar to the structure of the first continuum structure 211 as shown in FIG. 11B. As shown in FIG. 11A, the second continuum structure may comprise a second basal disk 2121, a plurality of second spacer disks (e.g., second spacer disks 2122 shown in FIG. 11A) and a plurality of second structural backbones (e.g., second structural backbones 2123 shown in FIG. 11A). The plurality of second structural backbones 2123 may pass through the plurality of second spacer disks 2122 and the second basal disk 2121. The proximal ends of the plurality of second structural backbones 2123 may be configured to receive push or pull driving to drive the second continuum structure to move. As shown in FIG. 11A, the first continuum structure 211 is disposed at the distal end of the second continuum structure, and the plurality of first structural backbones 2113 pass through the plurality of second spacer disks 2122 and the second basal disk 2121. In some embodiments, as shown in FIG. 11A, the second continuum structure 212 may also comprise a second fixing disk 2124. The distal ends of the plurality of second structural backbones 2123 are fixedly connected to the second fixing disk 2124.

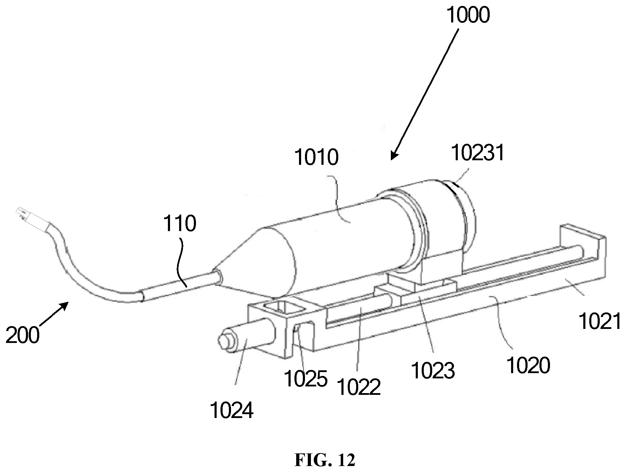

The proximal ends of the plurality of first structural backbones 2113 and the plurality of second structural backbones 2123 may be connected to a driving device. FIG. 12 shows a structural schematic diagram of a driving device 1000 according to some embodiments of the present disclosure. In some embodiments, the driving device 1000 may comprise a first driving mechanism 1010. As shown in FIG. 12, the first driving mechanism 1010 is connected to a proximal end of the auxiliary surgical instrument 200. In some embodiments, a plurality of first structural backbones (not shown in FIG. 12, such as the first structural backbones 2113 shown in FIG. 11A) and/or a plurality of second structural backbones (not shown in FIG. 12, such as the second structural backbone 2123 shown in FIG. 11A) may pass through the plurality of second spacer disks 2122 and the second basal disk 2121 and connect to the first driving mechanism 1010. The first driving mechanism 1010 may drive the first continuum structure 211 to bend in different directions in the space by pushing and pulling the plurality of first structural backbones 2113, and may drive the second continuum structure to bend in different directions in the space by pushing and pulling the plurality of second structural backbones 2123. For example, the first driving mechanism 1010 may comprise a plurality of double-ended screw assemblies, each double-ended screw assembly may comprise a double-ended screw and a pair of sliding blocks threaded to two threaded sections of the double-ended screw. The double-ended screw can be driven to rotate so as to drive a pair of sliding blocks to move towards opposite directions at the same speed. A pair of sliding blocks can be connected with a pair of symmetrical first structural backbones 2113 or second structural backbones 2123, so as to push and pull a pair of symmetrical first structural backbones 2113 or second structural backbones 2123 to drive the first continuum structure 211 or second continuum structure 212 to bend. For example, the first driving mechanism 1010 may comprise a proximal continuum structure, and the first continuum structure 211 or the second continuum structure may be connected to the proximal continuum structure to form a coupled dual continuum structure. The proximal continuum structure can be driven to bend through the double-ended screw assembly, so as to drive the first continuum structure 211 or the second continuum structure 212 to bend.

In some embodiments, as shown in FIG. 12, the driving device may also comprise a second driving mechanism 1020, which is connected to the instrument stem (e.g., the instrument stem 210 shown in FIG. 11A) of the auxiliary surgical instrument 200 via the first driving mechanism 1010. The driving device may be configured to drive the instrument stem 210 to feed or retreat, so as to drive the auxiliary surgical instrument 200 to feed or retreat in the patient's body and drive the ultrasonic surgical tool 100 to feed or retreat in the patient's body; or to drive the auxiliary surgical instrument 200 to enter or exit the patient's body in the case where the auxiliary surgical instrument 200 is not connected to the ultrasonic surgical tool 100. In some embodiments, the second driving mechanism 1020 may be a linear driving mechanism to drive the instrument stem 210 for linear movement. In some embodiments, the second driving mechanism 1020 may comprise a pedestal and a drive portion, the pedestal may be configured to support the first driving mechanism 1010, and the drive portion may be configured to drive the pedestal forward or backward. In some embodiments, the second driving mechanism 1020 may comprise a bracket 1021 with a sliding groove, on which a lead screw 1022 is rotatably disposed. The lead screw 1022 is sleeved with a slider 1023 as a pedestal, and the slider 1023 is in threaded connection with the lead screw 1022 and is slidingly disposed in the sliding groove of the bracket 1021. One end of the bracket 1021 can be provided with a motor 1024 as a second driving unit, and the output shaft of the motor 1024 can be fixedly connected with the lead screw 1022 through a coupling 1025. In some embodiments, the slider 1023 may also comprise a sleeve 10231 for mounting the continuum structure 211. The sleeve 10231 may be disposed on the slider 1023, or the sleeve 10231 may be integrally formed with the slider 1023. The motor 1024 may drive the lead screw 1022 so as to drive the slider 1023 and the sleeve 10231 to move linearly along the sliding groove, thereby enabling the feed motion of the instrument stem 210 and the auxiliary surgical instrument 200 disposed on the instrument stem 210. It may be understood by those skilled in the art that the second driving mechanism 1020 is not limited to the structure described above. Any driving mechanism capable of achieving the feed motion of surgical tools does not depart from the scope of the present disclosure.

As shown in FIG. 11A, the plurality of second spacer disks 2122 may be disposed at intervals to enhance the stability of the plurality of second structural backbones 2123 when being pushed or pulled. Similar to the first continuum structure 211, the second continuum structure may comprise any appropriate number of second spacer disks 2122.

In some embodiments, the shapes of the second basal disk 2121, the second spacer disks 2122, and the second fixing disk 2124 may be suitable structures such as an annular structure, a disk-shaped structure, and the like, and the cross section may be various shapes such as a circle, a rectangle, a polygon, and the like.

In some embodiments, the instrument stem 210 may also comprise a first straight rod segment 213 disposed between the first continuum structure 211 and the second continuum structure. In some embodiments, as shown in FIG. 11A, the second fixing disk 2124 of the second continuum structure may be fixedly connected to the proximal end of the first straight rod segment 213. In some embodiments, the instrument stem 210 may also comprise a second straight rod segment 214 connected to the proximal end of the second continuum structure. For example, in a laparoscopic surgical robot system, the auxiliary surgical tool 200 may extend into the patient's body through an opening (such as an incision or a natural opening, etc.) during surgical operations with the auxiliary surgical tool 200, and the second straight rod segment 214 may pass through the opening.

It may be understood by those skilled in the art that the structure for increasing the degree of freedom of the instrument stem 210 is not limited to continuum structures, but may also be a snake bone structure, a combined structure of a rod and a joint, and the like.

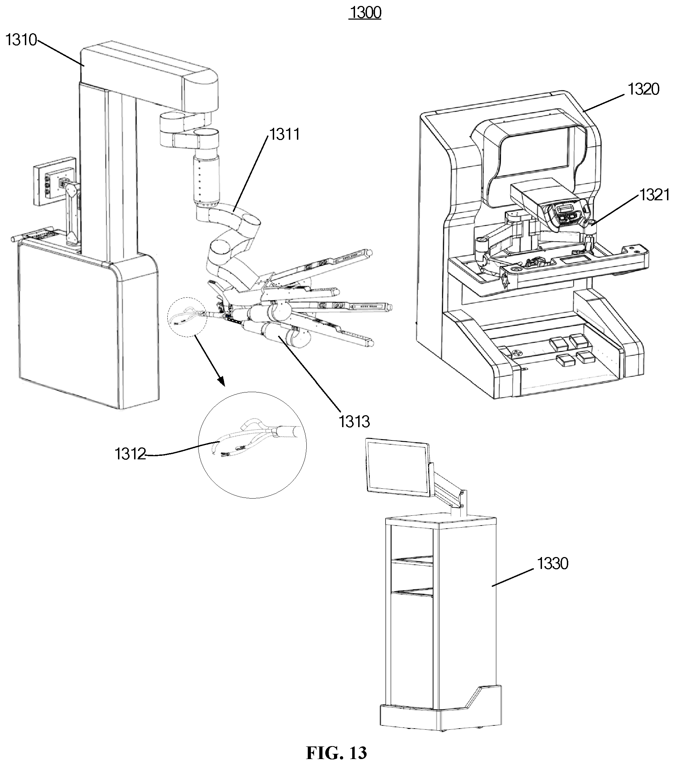

Some embodiments of the present disclosure also provide a surgical robot system. FIG. 13 shows a schematic diagram of a surgical robot system 1300 according to another embodiment of the present disclosure. As shown in FIG. 13, the surgical robot system 1300 may comprise an operation trolley 1310. The operation trolley 1310 may comprise at least one robotic arm 1311 and an assemblable ultrasonic surgical tool assembly (e.g., an assemblable ultrasonic surgical tool assembly comprised of an ultrasonic surgical tool 100 and an auxiliary surgical instrument 200) as in any of some embodiments of the present disclosure. At least one robotic arm 1311 may be a positioning arm of the surgical robot system as shown in FIG. 13. At least one robotic arm 1311 of the operation trolley 1310 may carry at least one surgical tool 1312 (e.g., a clamp, a curved scissors, or the like). An auxiliary surgical instrument of the assemblable ultrasonic surgical tool assembly (e.g., auxiliary surgical instrument 200) may be disposed at the distal end of the at least one robotic arm 1311. In some embodiments, the position and pose of the auxiliary surgical instrument 200 of the assemblable ultrasonic surgical tool assembly can be adjusted by controlling the movement of the at least one robotic arm 1311, thereby adjusting the position and pose of the ultrasonic surgical tool of the assemblable ultrasonic surgical tool assembly (not shown in the figure, for example, ultrasonic surgical tool 100).

In some embodiments, the surgical robot system 1300 may also comprise a surgeon console 1320. The operation trolley 1310 and the surgeon console 1320 can be connected by wired transmission or wireless transmission. In an operation, the user may control the operation of a surgical tool (e.g., auxiliary surgical instrument 200, clamp, curved scissors, etc.) and/or an imaging tool (e.g., endoscope) comprised in the operation trolley 1310 by operating the master manipulator 1321 of the surgeon console 1320. The operation trolley 1310 may usually be located on the patient's side and respond to control commands from the surgeon console 1320 to perform surgical operations on the patient. In some embodiments, a user can also control the opening and closing of the jaw body 152 of the ultrasonic surgical tool 100 of the assemblable ultrasonic surgical tool assembly by operating the master manipulator 1321.

In some embodiments, the assemblable ultrasonic surgical tool assembly may also comprise a clamp applicator (not shown). The clamp applicator may be disposed at a distal end of at least one robotic arm 1311. In some embodiments, the position and pose of the clamp applicator of the assemblable ultrasonic surgical tool assembly may be adjusted by controlling the movement of the at least one robotic arm 1311.

In some embodiments, the surgical robot system 1300 may also comprise a device trolley 1330. The device trolley 1330 may comprise a power source (not shown), the power source may be configured to connect with an ultrasonic surgical tool (e.g., ultrasonic surgical tool 100) of the assemblable ultrasonic surgical tool assembly to provide energy to an ultrasonic transducer (e.g., ultrasonic transducer 120 shown in FIG. 3) of the ultrasonic surgical tool 100. In some embodiments, the power source may provide a high-frequency voltage to the ultrasonic transducer section of the ultrasonic transducer. Due to the inverse piezoelectric effect, the ultrasonic transducer section may perform high-frequency vibration along the thickness direction, the high-frequency vibration may be transmitted to the ultrasonic blade head disposed at the distal end of the ultrasonic surgical tool 100. This may allow the ultrasonic blade head of the ultrasonic surgical tool 100 to perform a cutting or coagulation operation on the biological tissue.

In some embodiments, the operation trolley 1310 of the surgical robot system 1300 may also comprise at least one driving device 1313. At least one driving device 1313 may be disposed between at least one surgical tool 1312 and at least one robotic arm 1311. As shown in FIG. 13, the operation trolley 1310 may comprise a single robotic arm 1311, and a plurality of driving devices 1313 may be disposed on the robotic arm 1311. It may be understood by those skilled in the art that the operation trolley may also comprise a plurality of robotic arms. At least one driving device 1313 may comprise a sliding block driving device for driving the sliding block 223 of the auxiliary surgical instrument 200 to slide (thereby effecting the opening and closing of the jaw body 152 of the ultrasonic surgical tool 100) and/or a continuum structure driving device for driving the movement of the first continuum structure (such as the first continuum structure 211 shown in FIG. 11A) and the second continuum structure (such as the second continuum structure 212 shown in FIG. 11A).

It may be understood by those skilled in the art that the surgical robot system 1300 provided in this embodiment may be any suitable surgical robot including a laparoscopic surgical robot system.

In some embodiments, for the ultrasonic surgical tool 100, due to the inverse piezoelectric effect, the high-frequency AC voltage may be input to the ultrasonic transducer 120, thereby causing the ultrasonic transducer 120 to output a high-frequency vibration, which is transmitted to the head of the ultrasonic blade assembly 110 through the ultrasonic blade assembly 110. The head of the high-frequency vibrating ultrasonic blade may contactethe biological tissue, which has mechanical cutting function to the biological tissue on one hand, and the internal energy loss of the biological tissue under the action of the high-frequency vibration may make the biological tissue generate heat to generate coagulation on the other hand. The ultrasonic surgical tool 100 has a relatively small tissue damage range when performing cutting. There is no current passing through the human body in the operation, and there is no smoke and little scab, so it has higher safety.

When performing laparoscopic operation through an operation robot system, surgical instruments may usually enter the patient's body through a trocar. In order to enable the ultrasonic surgical tool to enter the trocar, it is necessary to limit the size of the ultrasonic surgical tool, especially the size of the ultrasonic transducer with large volume in the ultrasonic surgical tool. In general, the diameter of the ultrasonic transducer capable of passing through the trocar needs to be less than 8 mm and the length needs to be less than 40 mm. The upper limit of the output power of the ultrasonic transducer is positively correlated with the volume of the piezoelectric ceramic of the ultrasonic transducer (the upper limit of the output power = the power density of the piezoelectric ceramic * the volume of the piezoelectric ceramic). Therefore, the ultrasonic surgical tool used by surgical robot systems may generally comprise a micro ultrasonic transducer. The volume of piezoelectric ceramic may be small, so the output power may be limited, and the efficiency of tissue cutting may be low. The assemblable ultrasonic surgical tool assembly provided by the present disclosure can be pre-placed in patient's body before setting the trocar, and the ultrasonic surgical tool does not need to occupy the channel for surgical instruments to enter patient's body provided by the trocar. Based on this, the ultrasonic transducer of the ultrasonic surgical tool can comprise piezoelectric ceramic with large size and large volume (for example, a piezoelectric ceramic plate with an outer diameter of 12 mm and a thickness of 2.5 mm), which is beneficial for improving the output power of the ultrasonic transducer.

In some embodiments, the instrument stem of the auxiliary surgical instrument of the assemblable ultrasonic surgical tool assembly may comprise at least one continuum structure. Based on this, the flexibility of the auxiliary surgical instrument can be increased, thereby enhancing the flexibility of the ultrasonic surgical tool to perform surgical operations in patient's body.

In some embodiments, the pad may be sealingly connected to the proximal end of the ultrasonic transducer shell. Based on this, it may prevent a plurality of piezoelectric ceramic plates and a plurality of electrode slices of the ultrasonic transducer from being contaminated, which is conducive to the reuse of the ultrasonic transducer. On the other hand, it may avoid affecting the electrical connection of the electrode slices, thereby facilitating the improvement of the safety of the ultrasonic surgical tool 100. In addition, the pad may hold a plurality of wires used to transmit energy to a plurality of electrode slices of the ultrasonic transducer, thereby helping to avoid pulling the parts of the plurality of wires connected in the ultrasonic transducer during the control of the movement of the ultrasonic surgical tool, which is conducive to improving the stability of the ultrasonic surgical tool.

In some embodiments, the ultrasonic blade of the ultrasonic surgical tool (e.g., the ultrasonic blade assembly 110 of the ultrasonic surgical tool 100) is detachably connected (e.g., threaded) with the ultrasonic transducer (e.g., the ultrasonic transducer 120 of the ultrasonic surgical tool 100). Based on this, the ultrasonic blade can be disposable, and the ultrasonic transducer can be used for multiple times. In some embodiments, as shown in FIG. 4A, FIG. 4B, or FIG. 5, in the ultrasonic surgical tool 100, the ultrasonic blade assembly 110, the clamping assembly 130 connected with the ultrasonic blade assembly 110, the clamping torsional spring 140 sleeved on the ultrasonic blade shell and the jaw assembly 150 disposed at the distal end of the clamping assembly 130 may be disposable. And the disposable portion may be replaced by disassembling the connection between the ultrasonic blade assembly 110 and the ultrasonic transducer 120. Based on this, it is convenient to perform the operation of replacing consumables.

Note that the above are only exemplary embodiments of the present disclosure and the applied technical principles. Those skilled in the art would appreciate that the present disclosure is not limited to specific embodiments herein, and those skilled in the art could make various apparent changes, readjustments and substitutions without departing from the scope of protection of the present disclosure. Thus, although the present disclosure is described in more detail by the above embodiments, the present disclosure is not limited to the above embodiments. Without departing from the concept of the present disclosure, more other equivalent embodiments may be comprised, and the scope of the present disclosure is determined by the scope of the appended claims.

## Claims

1. An ultrasonic surgical tool, comprising:
an ultrasonic blade assembly, disposed at a distal end of the ultrasonic surgical tool, the ultrasonic blade assembly comprising an ultrasonic blade;
an ultrasonic transducer, coupled to a proximal end of the ultrasonic blade to output vibration to the ultrasonic blade; and
a clamping assembly, connected with the ultrasonic blade assembly, configured to mount or demount the ultrasonic surgical tool.

2. The ultrasonic surgical tool according to claim 1, wherein the ultrasonic blade comprises:
a proximal section, coupled to the ultrasonic transducer;
a distal section, having a transverse dimension less than a transverse dimension of the proximal section;and
a flange, disposed between the proximal section and the distal section;
wherein the ultrasonic blade assembly further comprises:
an ultrasonic blade shell, covering at least a portion of the proximal section and the distal section of the ultrasonic blade, and engaged with the flange;
wherein the distal section of the ultrasonic blade extends distally out of the ultrasonic blade shell, a distal end of the distal section forms an ultrasonic blade head.

3. The ultrasonic surgical tool according to claim 2, wherein the ultrasonic blade shell comprises:
a proximal ultrasonic blade shell, covering at least a portion of the proximal section of the ultrasonic blade; and
a distal ultrasonic blade shell, covering at least a portion of the distal section of the ultrasonic blade, the distal ultrasonic blade shell and the proximal ultrasonic blade shell being detachably connected;
wherein a connection part of the proximal ultrasonic blade shell and the distal ultrasonic blade shell is engaged with the flange.

4. The ultrasonic surgical tool according to claim 3, wherein the clamping assembly comprises:
a fixed clamp, fixedly connected with the distal ultrasonic blade shell; and
a movable clamp, rotatably connected with the distal ultrasonic blade shell and cooperated with the fixed clamp.

5. The ultrasonic surgical tool according to claim 4, wherein,
the fixed clamp comprises an upper fixed clamping ear and a lower fixed clamping ear; and
the movable clamp comprises an upper movable clamping ear and a lower movable clamping ear, and the upper fixed clamping ear cooperating with the upper movable clamping ear, the lower fixed clamping ear cooperating with the lower movable clamping ear.

6. The ultrasonic surgical tool according to claim 5, wherein further comprising:
a clamping torsional spring, at least a portion of the clamping torsional spring is sleeved on the ultrasonic blade shell, a distal end of the clamping torsional spring being fixedly connected with or propped against the upper movable clamping ear of the movable clamp of the clamping assembly, a proximal end of the clamping torsional spring being fixedly connected with the proximal ultrasonic blade shell, the clamping torsional spring being configured to apply a torque to the upper movable clamping ear to make the upper movable clamping ear tend away from the upper fixed clamping ear, thereby causing the lower movable clamping ear and the lower fixed clamping ear to tend to close.

7. The ultrasonic surgical tool according to claim 5 or 6, wherein,
the lower fixed clamping ear comprises a dent or a hole for mounting; and
the lower movable clamping ear comprises a dent or a hole for mounting; and/or the upper fixed clamping ear comprises a dent or a hole for engaging with a clamp applicator; and
the upper movable clamping ear comprises a dent or a hole for engaging with a clamp applicator.

8. The ultrasonic surgical tool according to claim 1 to 7, wherein further comprising:
a jaw assembly, disposed at a distal end of the clamping assembly, comprising:
a jaw base, connected to the distal end of the clamping assembly; and
a jaw body, disposed at a distal end of the jaw base, a proximal end of the jaw body being hinged to the jaw base.

9. The ultrasonic surgical tool according to claim 8, wherein,
the jaw base comprises:
a first sliding groove, provided on a first side of the jaw base; and
a second sliding groove, provided on a second side of the jaw base, the first sliding groove and the second sliding groove being oppositely disposed;
the jaw assembly further comprises:
at least one connecting rod, a distal end of the at least one connecting rod being hinged to the proximal end of the jaw body; and
a pin structure, hinged to a proximal end of the at least one connecting rod, two ends of the pin structure being slidingly connected with the first sliding groove and the second sliding groove, respectively.

10. The ultrasonic surgical tool according to claim 8 or 9, wherein the jaw assembly further comprises:
a jaw torsional spring, disposed between the jaw base and the jaw body, the jaw torsional spring being configured to apply a torque to the jaw body to make the jaw body tend to be closed.

11. The ultrasonic surgical tool according to any one of claims 1 to 10, wherein the ultrasonic transducer comprises:
a front plate, disposed at the distal end of the ultrasonic transducer;
a rear plate, disposed at the proximal end of the ultrasonic transducer; and
an ultrasonic transducer section, disposed between the front plate and the rear plate, the ultrasonic transducer section comprising:
a plurality of piezoelectric ceramic plates; and
a plurality of electrode slices, disposed between the front plate and the ultrasonic transducer section, between the plurality of piezoelectric ceramic plates, and between the ultrasonic transducer section and the rear plate;
wherein a first amplitude node of the ultrasonic surgical tool is located in the ultrasonic transducer section; and/ or
wherein a second amplitude node of the ultrasonic surgical tool is located at the flange.

12. The ultrasonic surgical tool according to claim 11, wherein the ultrasonic transducer further comprises:
an ultrasonic transducer shell, sleeved on the front plate, the ultrasonic transducer section and the rear plate, the ultrasonic transducer shell comprising a bump disposed at a proximal end of the ultrasonic transducer shell and propped against the proximal end of the rear plate; and
a fastener, an outer circumferential surface of the fastener being in threaded connection with an inner circumferential surface of a distal end of the ultrasonic transducer shell, a proximal end of the fastener being propped against a distal end of the front plate;
wherein the proximal section of the ultrasonic blade comprises a blind hole located on a central axis of the proximal section, the front plate comprises a columnar structure disposed at the distal end of the front plate and located on a central axis of the front plate, at least a portion of the proximal section of the ultrasonic blade extends into the fastener, an inner circumferential surface of the blind hole is in threaded connection with an outer circumferential surface of the columnar structure.

13. An assemblable ultrasonic surgical tool assembly, comprising:
the ultrasonic surgical tool according to any one of claims 1 to 12; and
an auxiliary surgical instrument, detachably connected with the ultrasonic surgical tool, configured to drive the ultrasonic surgical tool to move, the auxiliary surgical instrument comprising:
an instrument stem; and
an instrument end effector, disposed at a distal end of the instrument stem, the instrument end effector being detachably connected with the clamping assembly of the ultrasonic surgical tool.

14. The assemblable ultrasonic surgical tool assembly according to claim 13, wherein the instrument end effector of the auxiliary surgical instrument comprises:
a connecting part, the connecting part comprising a connecting column extending to the distal end of the instrument stem and a first bump structure and a second bump structure disposed on two sides of the connecting column, the first bump structure and the second bump structure being configured to connect with the ultrasonic surgical tool.

15. The assemblable ultrasonic surgical tool assembly according to claim 13 or 14, wherein the instrument end effector comprises:
a body, the body comprising a sliding space and a third sliding groove and a fourth sliding groove located at two opposite sides of the sliding space; and
a sliding block, the sliding block slidingly connected with the third sliding groove and the fourth sliding groove through at least one pin or bump, the sliding block comprising:
at least one groove structure, provided on an upper part of the sliding block and disposed above the sliding space, the at least one groove structure being configured to connect with the ultrasonic surgical tool.

16. The assemblable ultrasonic surgical tool assembly according to claim 15, wherein,
the body comprises a first arm and a second arm, the first arm and the second arm being oppositely disposed on two sides of the sliding space and extending to the distal end of the instrument stem, the first arm comprising the third sliding groove, the second arm comprising the fourth sliding groove.

17. The assemblable ultrasonic surgical tool assembly according to claim 15 or 16, wherein the auxiliary surgical instrument comprises:
a driving wire, passing through the instrument stem of the auxiliary surgical instrument, and a distal end of the driving wire being connected with the sliding block, the driving wire being configured to drive the sliding block to slide along the third sliding groove and the fourth sliding groove.

18. The assemblable ultrasonic surgical tool assembly according to any one of claims 13 to 17, wherein the instrument stem comprises:
a first continuum structure, the first continuum structure comprising:
a first basal disk, a plurality of first spacer disks and a plurality of first structural backbones, the plurality of first structural backbones passing through the plurality of first spacer disks and the first basal disk, proximal ends of the plurality of first structural backbones being configured to receive push or pull driving to drive the first continuum structure to move; and
a second continuum structure, the second continuum structure comprising:
a second basal disk, a plurality of second spacer disks and a plurality of second structural backbones, the plurality of second structural backbones passing through the plurality of second spacer disks and the second basal disk, proximal ends of the plurality of second structural backbones being configured to receive push or pull driving to drive the second continuum structure to move, wherein the first continuum structure is disposed at the distal end of the second continuum structure, the plurality of first structural backbones pass through the plurality of second spacer disks and the second basal disk.

19. The assemblable ultrasonic surgical tool assembly according to any one of claims 13 to 17, wherein further comprising:
a clamp applicator, the clamp applicator comprising:
a first clamp body and a second clamp body, a distal end of the first clamp body being provided with a third bump structure, a distal end of the second clamp body being provided with a fourth bump structure.

20. A surgical robot system, comprising:
an operation trolley, comprising at least one robotic arm; and
the assemblable ultrasonic surgical tool assembly according to any one of claims 13 to 19, wherein the auxiliary surgical instrument of the assemblable ultrasonic surgical tool assembly is disposed at a distal end of the at least one robotic arm.
